# EUROPÄISCHE PATENTSCHRIFT

(11) **EP 2 376 074 B1**
(45) Veröffentlichungstag und Bekanntmachung des Hinweises auf die Patenterteilung: **13.03.2019**
(21) Anmeldenummer: 10706476.8
(22) Anmeldetag: 08.01.2010
(51) Int. Cl.: A61K 31/155, C07C 257/10, C07D 309/28, C07C 279/18, C07C 257/18

(54) **VERFAHREN ZUR VERBESSERTEN BIOAKTIVIERUNG VON ARZNEISTOFFEN**
METHOD FOR IMPROVED BIOACTIVATION OF MEDICATIONS
PROCÉDÉ POUR AMÉLIORER LA BIOACTIVATION DE SUBSTANCES PHARMACEUTIQUES

(30) Priorität: 09.01.2009 DE 102009004204
(43) Veröffentlichungstag der Anmeldung: 19.10.2011
(73) Patentinhaber: Dritte Patentportfolio Beteiligungsgesellschaft mbH & Co. KG, 12529 Schönefeld/OT Waltersdorf (DE)
(72) Erfinder: CLEMENT, Bernd, 24106 Kiel (DE); SCHADE, Dennis, 24114 Kiel (DE)
(74) Vertreter: Zech, Stefan Markus
(86) Internationale Anmeldenummer: PCT/DE2010/000009
(87) Internationale Veröffentlichungsnummer: WO 2010/078867

(56) Entgegenhaltungen:
- EP-A1- 2 009 006
- EP-A2- 1 561 463
- WO-A1-2006/045514
- WO-A1-2008/009264
- WO-A1-2009/092358
- WO-A1-2009/095499
- WO-A2-2009/095503
- DE-A1- 2 640 484
- US-A- 5 225 408
- US-A- 5 587 372
- US-A- 5 610 144
- US-A- 5 840 758
- US-A1- 2002 019 437
- US-A1- 2007 093 432
- REHSE ET AL: "New NO Donors with Antithrombotic and Vasodilating Activities, Part 26 Amidoximes and Their Prodrugs" ARCHIV DER PHARMAZIE, VCH VERLAGSGESELLSCHAFT MBH, WEINHEIM, DE LNKD- DOI:10.1002/(SICI)1521-4184(199812)331:12< 375::AID-ARDP375>3.0.CO;2-F, Bd. 331, Nr. 12, 1. Januar 1998 (1998-01-01), Seiten 375-379, XP009132776 ISSN: 0365-6233
- FROEHLICH A K ET AL: "Metabolism of benzamidoxime (N-hydroxyamidine) in human hepatocytes and role of UDP-glucuronosyltransferases" XENOBIOTICA, Bd. 35, Nr. 1, Januar 2005 (2005-01), Seiten 17-25, XP009132770 ISSN: 0049-8254
- RIGGS J R ET AL: "Factor VIIa inhibitors: A prodrug strategy to improve oral bioavailability" BIOORGANIC & MEDICINAL CHEMISTRY LETTERS, PERGAMON, ELSEVIER SCIENCE, GB LNKD- DOI:10.1016/J.BMCL.2006.01.039, Bd. 16, Nr. 8, 15. April 2006 (2006-04-15) , Seiten 2224-2228, XP025106943 ISSN: 0960-894X [gefunden am 2006-04-15]
- ANSEDE J H ET AL: "In vitro metabolism of an orally active O-methyl amidoxime prodrug for the treatment of CNS trypanosomiasis" XENOBIOTICA, TAYLOR AND FRANCIS, LONDON, GB LNKD- DOI:10.1080/00498250500087671, Bd. 35, Nr. 3, 1. März 2005 (2005-03-01), Seiten 211-226, XP009132798 ISSN: 0049-8254
- CLEMENT B ET AL: "AMIDOXIMES OF PENTAMIDINE: SYNTHESIS, TRYPANOCIDAL AND LEISHMANICIDAL ACTIVITY" ARZNEIMITTEL FORSCHUNG. DRUG RESEARCH, ECV EDITIO CANTOR VERLAG, AULENDORF, DE, Bd. 35, Nr. 7, 1. Januar 1985 (1985-01-01) , Seiten 1009-1014, XP000566459 ISSN: 0004-4172
- SCHADE D ET AL: "The peptidylglycine a-amidating monooxygenase (PAM): A novel prodrug strategy for amidoximes and N-hydroxyguanidines?" CHEMMEDCHEM 2009 JOHN WILEY AND SONS LTD GBR LNKD- DOI:10.1002/CMDC.200900233, Bd. 4, Nr. 10, 1. Oktober 2009 (2009-10-01), Seiten 1595-1599, XP007912822
- SCHRODER A ET AL: "Arylazoamidoximes and related compounds as NO-modulators" ARCHIV DER PHARMAZIE 2010 WILEY-VCH VERLAG DEU LNKD- DOI:10.1002/ARDP.200900060, Bd. 343, Nr. 1, Januar 2010 (2010-01), Seiten 9-16, XP007912819
- FRANZ ESSER ET AL: "Cyclic guanidines. Part 6. Synthesis of benzimidazoles by intramolecular vicarious nucleophilic substitution of hydrogen" JOURNAL OF THE CHEMICAL SOCIETY, PERKIN TRANSACTIONS 1, CHEMICAL SOCIETY, LETCHWORTH; GB LNKD- DOI:10.1039/A900553F, 1. Januar 1999 (1999-01-01), Seiten 1153-1154, XP007912863 ISSN: 0300-922X
- PETERLIN-MASIC L ET AL: "Metabolism-Directed Optimisation of Antithrombotics: The Prodrug Principle" CURRENT PHARMACEUTICAL DESIGN, BENTHAM SCIENCE PUBLISHERS, NL LNKD- DOI:10.2174/138161206775193172, Bd. 12, Nr. 1, 1. Januar 2006 (2006-01-01) , Seiten 73-91, XP009132773 ISSN: 1381-6128
- MARRIOTT HELEN M ET AL: "Decreased alveolar macrophage apoptosis is associated with increased pulmonary inflammation in a murine model of pneumococcal pneumonia" JOURNAL OF IMMUNOLOGY, AMERICAN ASSOCIATION OF IMMUNOLOGISTS, US, Bd. 177, Nr. 9, 1. November 2006 (2006-11-01), Seiten 6480-6488, XP007912841 ISSN: 0022-1767
- WELLER H N ET AL: "SYNTHESIS OF N-ALKYL-1,2,4-OXADIAZINONES AS ANGIOTENSIN-II (AT1) RECEPTOR ANTAGONISTS", HETEROCYCLES.INTERNATIONAL JOURNAL FOR REVIEWS AND COMMUNICATIONS IN HETEROCYCLIC CHEMISTRY, ELSEVIER SCIENCE PUBLISHERS B.V. AMSTERDAM, NL, vol. 36, no. 5, 1 January 1993 (1993-01-01), pages 1027-1038, XP001007292, ISSN: 0385-5414

## Beschreibung

Die vorliegende Erfindung betrifft ein Verfahren zur Verbesserung der Bioaktivität von Arzneistoffen.

Die Voraussetzung für einen therapeutischen Effekt eines Arzneistoffes nach oraler Gabe stellt dessen Aufnahme aus dem Gastrointestinaltrakt dar. Der wichtigste Mechanismus eines solchen Effektes ist die passive Diffusion. Der Grad der Resorption auf dem Weg der passiven Diffusion ist unter anderem abhängig von der Lipophilie.

Ein weiteres Problem bei der medikamentösen Behandlung vieler Krankheiten ist die Notwendigkeit, die Blut-Hirn-Schranke zu passieren. Die Blut-Hirn-Schranke stellt eine effektive Barriere in Bezug auf die Absorption von Substanzen ins Gehirn dar. Sie sichert die selektive Aufnahme und verhindert das Eindringen von Substanzen. Darüber hinaus fungiert die Blut-Hirn-Schranke nicht nur als physikalische, sondern auch als enzymatische Barriere. An der Penetration von Substanzen ins Gehirn sind verschiedene Prozesse beteiligt. Im Handel befinden sich im Vergleich zu anderen Indikationen nur wenige Arzneimittel, die im zentralen Nervensystem (ZNS) ihre Wirkung entfalten. Davon gelangt der überwiegende Teil durch Diffusion ins ZNS. Auf diesem Wege werden Krankheiten wie die Epilepsie, chronische Schmerzen oder Depressionen behandelt. Andere schwerwiegende Funktionsstörungen wie beispielsweise Hirntumore oder die Amyotrophe Lateralsklerose können auf diesem Wege heutzutage nur schwer behandelt werden.

Um Biomembranen durch passive Diffusion überwinden zu können, sollte eine Substanz lipophil sein, ein kleineres Molekulargewicht als 500 Da besitzen und sie sollte ungeladen vorliegen. Um spezifisch, kleine hochpolare Moleküle wie Aminosäuren oder Zucker zu absorbieren, sind verschiedene Transporter-Systeme, wie beispielsweise Nucleosid-Transporter, Influx- und Efflux-Transporter für organische Anionen oder Kationen, Glucose-Transporter, Peptid-Transporter und Aminosäure-Transporter an den Biomembranen mit Schrankenfunktion (Gastrointestinaltrakt, Blut-Hirn-Schranke) exprimiert.

Zur Verbesserung der pharmakokinetischen Eigenschaften werden daher verschiedene Prodrug-Systeme herangezogen. Bei einem Prodrug handelt es sich um einen pharmakologisch nicht oder nur sehr wenig aktiven Arzneistoff, der erst durch Metabolisierung im Organismus in einen aktiven Metaboliten überführt wird.

So offenbart die WO 2008/009264 A1 die Verbesserung der Bioverfügbarkeit von Arzneistoffen, die wenigstens eine Amidinfunktion aufweisen und entsprechend modifizierte Arzneistoffe enthaltende Arzneimittel.

N-Hydroxyamidine (Amidoxime) und N-Hydroxyguanidine stellen bekannte Prodrug-Prinzipien zur Erhöhung der oralen Bioverfügbarkeit der Amidine [Clement, B. Methoden zur Behandlung und Prophylaxe der Pneumocystis carinii Pneumonie (PCP) und anderen Erkrankungen sowie Verbindungen und Formulierungen zum Gebrauch bei besagten Methoden. [DE4321444] und Guanidine dar. Die Stickstoffatome der Amino und der Iminogruppe liegen bei den Salzen der Amidine und Guanidine im mesomeren Gleichgewicht vor, die Konzepte können auf beide Stickstoffatome angewendet werden.

Die Überführung in einen aktiven Metaboliten verläuft dabei, je nach zu Grunde liegendem Prodrug-Konzept, über unterschiedliche Enzymsysteme. Das in praktisch allen Formen des Lebens vorkommende Enzymsystem ist das Cytochrom-P450 (CYP450), das unter anderem folgende Reaktionen katalysiert:
N-Oxidation, S-Oxidation, N-Desalkylierung, O-Desalkylierung, S-Desalkylierung, Desaminierung, Dehalogenierung, sowie Hydroxylierung von Aromaten und Aliphaten.

Die Vielseitigkeit des Enzymsystems CYP450 bringt es mit sich, dass verschiedene Substrate und Arzneimittel bei Ihrem Umsatz um das System konkurrieren. Es kommt zu Interaktionen, Wechselwirkungen und unerwünschten gegenseitigen Beeinflussungen. Aus diesem Grund wird eine CYP450-unabhängige Bioaktivierung in der Prodrug-Entwicklung angestrebt.

Es ist daher Aufgabe der Erfindung, ein Prodrug-System zur Verfügung zu stellen, das einen Weg der Bioaktivierung nutzt, der unabhängig vom Cytochrom P450 (CYP450)-Enzymen ist. Diese Aufgabe wird durch die in den Ansprüchen beschriebenen Gegenstand gelöst. Die Unteransprüche geben vorteilhafte Ausgestaltungen der Erfindung an.

Erfindungsgemäß wird die Aufgabe in einem Aspekt durch ein Prodrug gemäß Anspruch 1 umfassend eine die allgemeine Formel (I) oder (II) aufweisende Teilstruktur 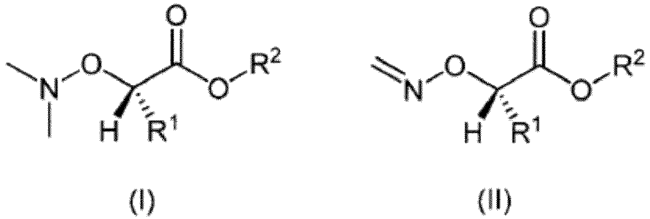 wobei R¹ und R² Wasserstoff, Alkyl- oder Arylreste sind gelöst

In einer bevorzugten Ausführungsform der Erfindung bedeutet der Begriff "Teilstruktur", wie hierin verwendet, dass das unter der entsprechenden Formel angegebene ein Strukturelement Teil der Formel eines Stoffes, bevorzugt eines Prodrugs ist. Beispielsweise stellt die Verbindung O-Carboxymethylbenzamidoxim (1) ein entsprechendes Prodrug des Arzneistoffes Benzamidin dar, wobei die Teilstruktur eine Teilstruktur der Formel (II) ist und R¹ und R² jeweils ein Wasserstoffatom sind. Diese Teilstruktur ist ein Substituent an einem Benzolring und stellt mit diesem zusammen den Arzneistoff Benzamidin dar.

In einer bevorzugten Ausführungsform der Erfindung bedeutet der Begriff "Prodrug", wie hierin verwendet, einen selbst inaktiven oder pharmakologisch nur sehr wenig aktiven Stoff, der erst durch Verstoffwechselung (Metabolisierung) im Organismus in einen Arzneistoff, der pharmakologisch aktiv ist, überfihrt wird. Das Prodrug kann, muss aber nicht eine verbesserte orale Bioverfügbarkeit als der eigentliche wirksame Arzneistoff aufweisen. Alternativ kann ein Prodrug verwendet werden, weil es im Vergleich zum Arzneistoff eine verbesserte Löslichkeit, Bioaktivierung, Blut-Hirn-Schrankengängigkeit, physikalisch-chemische Stabilität, eine geringere Toxizität und/oder einen tolerierbaren oder angenehmeren Geschmack aufweist. So wird das 2'-Ethyl-Succinat von Erythromycin A wegen des bitteren Geschmackes, nicht etwa wegen unzureichender Resorption oder Löslichkeit von Erythromycin A, als Prodrug an Kinder verabreicht (Bhadra et al. (2005), J. Med. Chem.).

In einer weiteren bevorzugten Ausführungsform der Erfindung wird das ursprüngliche Prodrug nicht in einer Einschrittreaktion vom Prodrug zum Arzneistoff metabolisiert, sondern über mehrere Reaktionsschritte, wobei jeder der aus einem Reaktionsschritt hervorgehenden Metaboliten gegenüber dem ursprünglichen Prodrug eine oder mehrere gleiche und/oder unterschiedliche vorteilhaftere Eigenschaften aufweisen kann. Dabei ist es möglich, dass nicht alle Metaboliten vorteilhafte Eigenschaften gegenüber dem Prodrug aufweisen. Beispielsweise kann ein erstes Verstoffwechselungsprodukt des Prodrugs eine gegenüber dem Prodrug erhöhte pharmakologische Aktivität aufweisen, ein aus dem ersten Verstoffwechselungsprodukt abgeleitetes zweites Verstoffwechselungsprodukt kann ebenfalls eine gegenüber dem Prodrug erhöhte pharmakologische Aktivität, und ein vom zweiten Verstoffwechselungsprodukt abgeleitetes drittes Verstoffwechselungsprodukt kann eine gegenüber dem Prodrug erhöhte Blut-Hirnschrankengängigkeit und physikalisch-chemische-Stabilität aufweisen.

In einer bevorzugten Ausführungsform der Erfindung wird unter dem Begriff "physikalisch-chemische Stabilität", wie hierin verwendet, die Eigenschaft eines Stoffes, beispielsweise eines Prodrugs oder eines Arzneistoffes verstanden, in Form einer relevanten wässrigen Lösung, beispielsweise in Wasser, einem Puffer oder eine physiologischen Kochsalzlösung gelöst, ohne chemische Zersetzung, beispielsweise Hydrolyse, gelagert und/oder verwendet werden zu können. In einer weiteren bevorzugten Ausführungsform der Erfindung bedeutet der Begriff, wie hierin verwendet, dass der Stoff in stabiler und synthetischer Form synthetisiert werden kann. In einer weiteren bevorzugten Ausführungsform der Erfindung bedeutet der Begriff, wie hierin verwendet, dass bei der Synthese des Stoffes einzelne, relevante Synthesevorstufen gegenüber analogen Produkten, Vorstufen oder Zwischenprodukte bei anderen Stoffen, die nach einer analogen oder gleichen Synthesestrategie hergestellt wurden, derart stabiler sind, dass nachfolgende Syntheseprodukte oder -zwischenprodukte in einer stabileren Form hergestellt oder überhaupt erst hergestellt werden können.

In einer Ausführungsform wird die Aufgabe durch ein Prodrug, dadurch gekennzeichnet, dass die vom Prodrug umfasste Teilstruktur Bestandteil eines Hydroxylamins, eines *N*-Oxids, eines Nitrons, eines Diazeniumdiolates (NONOat) oder eines ähnlichen N-O-haltigen Stickstoffmonoxid-Donors, einer Hydroxamsäure, eines Hydroxyharnstoffs, eines Oxims, eines Amidoxims (N-Hydroxyamidins), eines N-Hydroxyamidinohydrazons oder eines N-Hydroxyguanidins ist gelöst.

Beispielsweise ist die Teilstruktur im Falle des Prodrugs Carboxymethylbenzamidoxim (1) zum Arzneistoff Benzamidin eine Teilstruktur der Formel (II), RI und R2 sind jeweils ein Wasserstoffatom, und die vom Prodrug umfasste Teilstruktur ist Bestandteil eines Amidoxims (N-Hydroxyamidins).

Außerdem offenbart ist ein Prodrug, dadurch gekennzeichnet, dass das Prodrug zu einem Arzneistoff metabolisiert wird, der ein Arzneistoff zur Behandlung von mit einer Stickstoffmonoxid-Defizienz assoziierten Erkrankungen ist.

In einer Ausführungsform wird die Aufgabe durch ein Prodrug, dadurch gekennzeichnet, dass das Prodrug bzw. der entsprechende Arzneistoff aus der Gruppe ausgewählt ist, die Proteasehemmstoffe, der DNA- und RNA-interkalierende Verbindungen, Hemmstoffe viraler Enzyme und N-Methyl-D-Aspartat-Rezeptor-Antagonisten umfasst gelöst.

In einer bevorzugten Ausführungsform der vorliegenden Erfindung ist der Begriff "übergeordnete Teilstruktur", wie hierin verwendet, derart zu verstehen, dass diese übergeordnete Teilstruktur einerseits eine Teilstruktur der Formel (I) bzw. (II) umfasst und andererseits Teil der Gesamtstruktur des betreffenden Stoffes ist. Beispielsweise umfasst im Falle des Prodrugs Carboxymethylbenzamidoxim (1) zum Arzneistoff Benzamidin (2) die übergeordnete Teil-struktur, hier bezeichnet als (Ia), die Teilstruktur der Formel (IIa), wobei R¹ und R² Wasser-stoff sind, und die Teilstruktur, hier bezeichnet als (1b), ist die Teilstruktur der Formel (II), wobei R¹ und R² ebenfalls Wasserstoff sind gelöst.

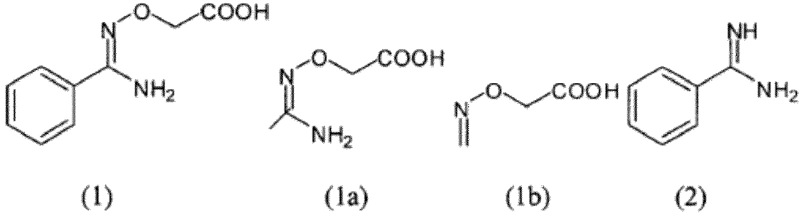

In einer Ausführungsform wird die Aufgabe durch ein Prodrug, dadurch gekennzeichnet, dass die Teilstruktur die allgemeine Formel IIa oder IIb 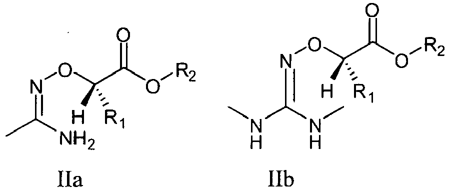 aufweist gelöst.

Beispielsweise umfasst im Falle des Prodrugs Carboxymethylbenzamidoxim (1) zum Arzneistoff Benzamidin die übergeordnete Teilstruktur die Teilstruktur der Formel (IIa), wobei R¹ und R² Wasserstoff sind, die Teilstruktur ist die Teilstruktur der Formel (II), wobei R¹ und R² ebenfalls Wasserstoff sind, und der Arzneistoff weist statt der Teilstruktur der Formel (IIa) im Prodrug die Struktur (IIa-1) auf.

In einer Ausführungsform wird die Aufgabe durch ein Prodrug, dadurch gekennzeichnet, dass das Prodrug ein Prodrug zu einem Arzneistoff ist, wobei die Teilstruktur der allgemeinen Formel IIa nach Metabolisierung eine die Formel 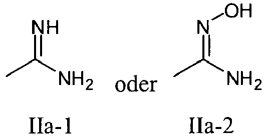 und die Teilstruktur der allgemeinen Formel IIb nach Metabolisierung eine die Formel 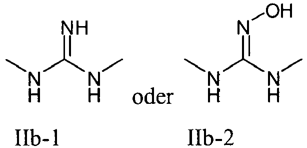 aufweisende Struktur besitzt gelöst.

In einem weiteren Aspekt der Erfindung wird die Aufgabe durch die Verwendung einer die allgemeine Formel (I) oder (II) ausbildende Teilstruktur 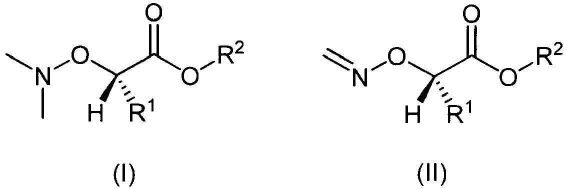 als Bestandteil der Gesamtstruktur eines Prodrugs, welches ein Prodrug zu einem Arzneistoff ist, wobei R¹ und R² Wasserstoff, Alkyl- oder Arylreste sind gelöst.

In einer Ausführungsform wird die Aufgabe durch die Verwendung eines Prodrugs, wobei die Teilstruktur die allgemeine Formel (II) aufweist, diese Teil einer übergeordneten Teilstruktur IIa bzw. IIb 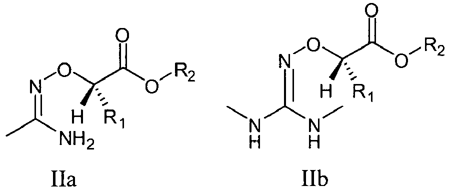 als Ersatz einer Amidin- bzw. Guanidin-Gruppe eines Arzneistoffes zur Verbesserung der Löslichkeit, oralen Bioverfügbarkeit, Blut-Hirn-Schrankengängigkeit, des Geschmacks und/oder der physikalisch-chemischen Stabilität gelöst.

In einer Ausführungsform wird die Aufgabe durch die Verwendung eine Prodrugs, wobei das Prodrug ein Prodrug zu einem Arzneistoff ist, der die gleiche Struktur wie das Prodrug aufweist, abgesehen davon, dass er statt der übergeordneten Teilstruktur IIa eine der Teilstrukturen IIa-1 oder IIa-2 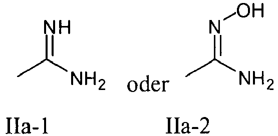 bzw. statt der übergeordneten Teilstruktur IIb eine der Teilstrukturen IIb-1 oder IIb-2 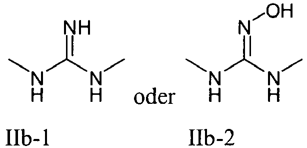 Aufweist, gelöst.

In einer Ausführungsform wird die Aufgabe durch die Verwendung eines Prodrugs zur Aktivierung des Arzneistoffs durch Peptidylglycin α-Amidierende Monooxygenase (PAM) gelöst.

In einer bevorzugten Ausführungsform der Erfindung bedeutet die Bezeichnung "Aktivierung des Prodrugs durch Peptidylglycin α-Amidierende Monooxygenase (PAM)", "Aktivierung eines Prodrugs durch den PAM-Aktivierungsweg", Bioaktivierung oder dergleichen, wie hierin verwendet, dass das Prodrug von PAM als Substrat erkannt und verstoffwechselt wird. In einer bevorzugten Ausführungsform der Erfindung bedeutet die Bezeichnung "Einschleusung eines Arzneistoffes in den PAM-Aktivierungsweg" umfassend Herstellung eines Prodrugs des Arzneistoffes", wie hierin verwendet, dass von einem in den PAM-Aktivierungsweg einzuschleusenden Arzneistoff eine entsprechende Prodrugform hergestellt wird, die von PAM erkannt und verstoffwechselt wird. In einer bevorzugten Ausführungsform liegt die Affinität des Prodrug zur PAM gegenüber dem Arzneistoff 1-1000-fach, 2-100-fach, 3-50-fach, 4-40-fach, 5-20-fach oder 6-15-fach höher, wie der Fachmann mit Hilfe der K_{M}-Werte ermitteln kann.

In einer Ausführungsform wird die Aufgabe durch die Verwendung eine Prodrugs, dadurch gekennzeichnet, dass die Teilstruktur Bestandteil eines Hydroxylamins, eines *N*-Oxids, eines Nitrons, eines Diazeniumdiolates (NONOat) oder eines ähnlichen N-O-haltigen Stickstoffmonoxid-Donors, einer Hydroxamsäure, eines Hydroxyharnstoffs, eines Oxims, eines Amidoxims (*N*-Hydroxyamidins), eines *N*-Hydroxyamidinohydrazons oder eines *N*-Hydroxyguanidins ist.

In einem weiteren Aspekt der Erfindung wird die Aufgabe durch ein Verfahren zur Einschleusung eines Arzneistoffes umfassend eine freie Amidin- oder Guanidinfunktion in den PAM-Aktivierungsweg, umfassend Herstellen eines Prodrugs des Arzneistoffes gelöst.

In einem weiteren Aspekt der Erfindung wird die Aufgabe durch ein Verfahren zur Behandlung eines Patienten, umfassend Verabreichen eines Prodrugs an den Patienten gelöst.

In einem weiteren Aspekt der Erfindung wird die Aufgabe durch die Verwendung eines Prodrugs zur Herstellung eines Arzneistoffes gelöst.

In einer bevorzugten Ausführungsform der Erfindung ist der Arzneistoff ein Arzneistoff bzw. das Prodrug ein Prodrug zur Bekämpfung viraler Infektionen wie Influenza, zur Bekämpfung von HIV-Infektionen, zur Prophylaxe und Behandlung der viszeralen und kutanen Leishmaniose, zur Prophylaxe der Pneumocystis carinii Pneumonie (PcP), zur Behandlung der Trypanosomiasis (afrikanische Schlafkrankheit), zur Behandlung der Malaria, zur Behandlung der Babesiose, zur Hemmung der Blutgerinnung, beispielsweise Primärprävention von venösen thromboembolischen Ereignissen, zur Schlaganfallprophylaxe bei Patienten mit Vorhofflimmern, zur Blutdrucksenkung, zur Wachstumshemmung maligner Tumore, zur Neuroprotektion, zur Bekämpfung viraler Infektionen wie Influenza, zur (diurethischen) Ausschwemmung von Wasser aus dem Körper, z.B. bei Herzinsuffizienz, Lungenödemen, Vergiftungen, Niereninsuffizienz oder Leberzirrhosen, zur Behandlung von Allergien, zur Behandlung von Asthma, zur Behandlung entzündlicher Erkrankungen, beispielsweise Rheuma oder Pankreatitis, zur Prophylaxe der Ischemie (Mangeldurchblutung).

Außerdem offenbart ist die Verwendung bzw. das Verfahren eine Verwendung bzw. ein Verfahren zur Behandlung von mit einer Stickstoffmonoxid-Defizienz assoziierten Erkrankungen ist.

In einer Ausführungsform wird die Aufgabe durch die Verwendung eines Prodrugs, dadurch gekennzeichnet, dass der Arzneistoff bzw. das Prodrug aus der Gruppe der Proteasehemmstoffe, der DNA- und RNA-interkalierenden Verbindungen, der Hemmstoffe viraler Enzyme und der N-Methyl-D-Aspartat-Rezeptor-Antagonisten ausgewählt ist gelöst.

In einer Ausführungsform wird die Aufgabe durch die Verwendung eines Prodrugs, wobei die Verwendung eine Verwendung zur Prophylaxe und/oder Therapie der viszeralen und/oder kutanen Leishmaniose, der Trypanosomiasis, der 2. Phase der Trypanosomiasis oder der durch Pneumocystis carinii verursachten Pneumonie, zur Wachstumshemmung maligner Tumoren, zur Blutgerinnungshemmung, zur Blutdrucksenkung, zur Neuroprotektion oder zur Bekämpfung von viralen Infektionen, einschließlich Influenza und HIV-Infektionen ist gelöst.

In einem weiteren Aspekt der Erfindung wird die Aufgabe durch einen Arzneistoff mit einer die allgemeine Formel (I) oder (II) aufweisenden Teilstruktur 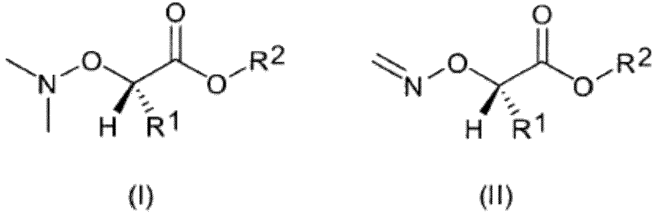 wobei R¹ und R² Wasserstoff, Alkyl- oder Arylreste sind gelöst.

In einer Ausführungsform wird die Aufgabe durch einen Arzneistoff mit einer die allgemeine Formel (I) oder (II) aufweisenden Teilstruktur, dadurch gekennzeichnet, dass die Teilstruktur Bestandteil eines Hydroxylamins, eines N-Oxids, eines Nitrons, eines Diazeniumdiolates (NONOat) oder eines ähnlichen N-O-haltigen Stickstoffmonoxid-Donors, einer Hydroxamsäure, eines Hydroxyharnstoffs, eines Oxims, eines Amidoxims (N-Hydroxyamidins), eines N-Hydroxyamidinohydrazons oder eines N-Hydroxyguanidins ist gelöst.

Zudem offenbart ist ein der Arzneistoff der zur Behandlung von mit einer Stickstoffmonoxid-Defizienz assoziierten Erkrankungen eingerichtet ist.

In einer Ausführungsform wird die Aufgabe durch einen Arzneistoff, dadurch gekennzeichnet, dass der Arzneistoff ausgewählt ist aus der Gruppe der Proteasehemmstoffe, der DNA- und RNA-interkalierenden Verbindungen, der Hemmstoffe viraler Enzyme und der N-Methyl-D-Aspartat-Rezeptor-Antagonisten gelöst.

In einem weiteren Aspekt der Erfindung wird die Aufgabe durch die Verwendung einer O-carboxyalkylierten N-0-haltigen Funktionalität zum Herstellen eines Arzneistoffs mit einer die allgemeine Formel (I) oder (II) ausbildenden Teilstruktur 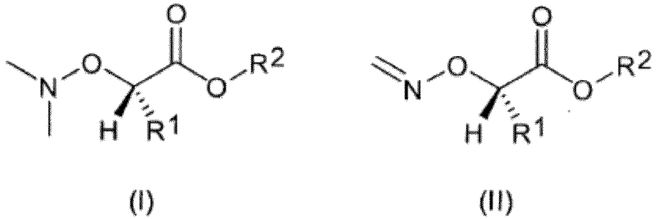 wobei R¹ und R² Wasserstoff, Alkyl- oder Arylreste sind, zur Verbesserung der Löslichkeit, Bioverfiigbarkeit, Blut-Himschrankengängigkeit, Bioaktivierung und/oder der physikalisch-chemischen Stabilität des Arzneistoffs gelöst.

In einer Ausführungsform wird die Aufgabe durch die Verwendung eines Arzneistoffs mit einer O-carboxyalkylierten N-O-haltigen Funktionalität zur Aktivierung des Arzneistoffs durch Peptidylglycin α-Amidierende Monooxygenase (PAM) gelöst.

In einer Ausführungsform wird die Aufgabe durch die Verwendung eines Arzneistoffs, dadurch gekennzeichnet, dass die Teilstruktur Bestandteil eines Hydroxylamins, eines N-Oxids, eines Nitrons, eines Diazeniumdiolates (NONOat) oder eines ähnlichen N-O-haltigen Stickstoffmonoxid-Donors, einer Hydroxamsäure, eines Hydroxyhamstoffs, eines Oxims, eines Amidoxims (N-Hydroxyamidins), eines N-Hydroxyamidinohydrazons oder eines N-Hydroxyguanidins ist gelöst.

Außerdem offenbart ist ein Arzneistoff, der zur Behandlung von mit einer Stickstoffmonoxid-Defizienz assoziierten Erkrankungen eingerichtet ist.

In einer Ausführungsform wird die Aufgabe durch die Verwendung eines Arzneistoffs, dadurch gekennzeichnet, dass der Arzneistoff ausgewählt ist aus der Gruppe der Proteasehemmstoffe, der DNA- und RNA-interkalierenden Verbindungen, der Hemmstoffe viraler Enzyme und der N-Methyl-D-Aspartat-Rezeptor-Antagonisten gelöst.

In einer Ausführungsform wird die Aufgabe durch die Verwendung eines Arzneistoffs, dadurch gekennzeichnet, dass der Arzneistoff zur Prophylaxe und/oder Therapie der viszeralen und/oder kutanen Leishmaniose, der Trypanosomiasis, der 2. Phase der Trypanosomiasis oder der durch Pneumocystis carinii verursachten Pneumonie, zur Wachstumshemmung maligner Tumoren, zur Blutgerinnungshemmung, zur Blutdrucksenkung, zur Neuroprotektion oder zur Bekämpfung von viralen Infektionen, einschließlich Influenza und HIV-Infektionen eingerichtet ist, gelöst.

In einem weiteren Aspekt der Erfindung werden pharmazeutische Verbindungen, pharmazeutische Zusammensetzungen und Arzneimittel bereitgestellt, die die erfindungsgemäßen Verbindungen und/oder ihre Salze umfassen. Vorzugsweise enthalten die pharmazeutischen Zusammensetzungen Träger und/oder Hilfsstoffe und sind idealer Weise pharmazeutisch verträglich. Derlei Träger und Hilfsstoffe sind dem Fachmann allgemein bekannt. Auch werden die erfindungsgemäßen Verbindungen zur Verwendung in der Medizin bereitgestellt.

Es ist ausreichend, wenn der Arzneistoff mindestens eine oder mehrere wirksame Amidin-, N-Hydroxyamidin- (Amidoxim), Guanidin- bzw. N-Hydroxyguanidin-Funktionen in der vorgeschlagenen Form enthält. Der Arzneistoff kann demnach z.B. mehrere Amidoxim-Funktionen (z.B. zwei wie bei dem Pentoximester) bzw. N-Hydroxyguanidin-Funktionen enthalten, wobei dann mindestens eine dieser Gruppen in der vorstehend beschrieben Art modifiziert ist. Genauso können auch Mischungen von Arzneistoffen eingesetzt werden von denen mindestens einer erfindungsgemäß modifiziert ist.

Die Verabreichung der erfindungsgemäßen Verbindungen kann einmalig, als Bolus-Verabreichung, jeden Tag, wöchentlich oder monatlich erfolgen. Der Weg der Verabreichung ist ebenfalls leicht zu bestimmen. Grundsätzlich kommt eine orale, rektale, parenterale wie intravenöse, intramuskuläre, subkutane, transdermale Gabe, intrapulmonale Verabreichung sowie Gabe als Aerosol, intravesikale Instillation, intraperitoneale oder intrakardiale Injektion, Aufnahme über Schleimhäute oder intravaginale Applikation zum Beispiel über Suppositorien in Betracht. Die orale Darreichungsform kann als flüssige, halbfeste oder feste Zubereitung, insbesondere als Tablette, Dragee, Pellet oder Mikrokapsel aufgemacht sein. Hierbei werden für solche Ausführungsformen, bei denen flüssige Zubereitungen eingesetzt werden, der Wirkstoff bzw. das Wirkstoffgemisch in einem geeigneten, nicht toxischen Lösungsmittel, wie zum Beispiel Wasser, einwertige Alkohole, insbesondere Ethanole, mehrwertige Alkohole, insbesondere Glycerin und/oder Propandiol, Polyglykole, insbesondere Polyethylenglykole und/oder Miglyol, Glycerinformal, Dimethylisosorbit, natürliche oder synthetische Öle, aufgenommen. Für die Herstellung von halbfesten oder festen Zubereitungen gelangen die üblichen Grundmassen, wie beispielsweise Bentonit, Veegum, Guarmehl und/oder Cellulosederivate, insbesondere Methylcellulose und/oder Carboxymethylcellulose, sowie Polymere aus Vinylalkoholen und/oder Vinylpyrrolidonen, Alginate, Pektine, Polyacrylate, feste und/oder flüssige Polyethylenglykole, Paraffine, Fettalkohole, Vaseline und/oder Wachse, Fettsäuren und/oder Fettsäureester zur Anwendung.

Des Weiteren können in festen Zubereitungen die an sich bekannten Streckmittel, wie beispielsweise kolloidale Kieselsäure, Talkum, Milchzucker, Stärkepulver, Zucker, Gelatine, Metalloxide und/oder Metallsalze enthalten sein. Als weitere Zusatzstoffe bieten sich Stabilisatoren, Emulgatoren, Dispergatoren sowie Konservierungsstoffe an.

Überraschenderweise hat sich gezeigt, dass *O*-carboxyalkylierte N-O-haltige Funktionalitäten der allgemeinen Formel (I) oder (II), die über die Bindungen am Stickstoff (N) an ein Arzneistoffmolekül gebunden sind, 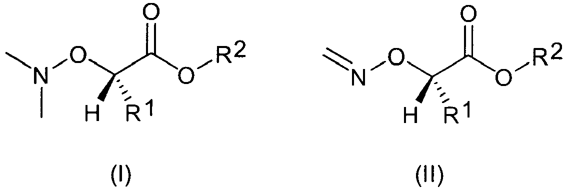 wobei (I) und (II) beispielsweise Bestandteil eines Hydroxylamins, eines *N*-Oxids, eines Nitrons, eines Diazeniumdiolates (NONOat) oder ähnlichen N-O-haltigen Stickstoffmonoxid-Donoren, einer Hydroxamsäure, eines Oxims, eines Amidoxims (*N*-Hydroxyamidins), eines *N*-Hydroxyamidinohydrazons oder eines *N*-Hydroxyguanidins sein können, und R¹ (welcher *pro*-*R*-konfiguriert sein muss) und R² Wasserstoff, Alkyl- oder Arylreste sein können, einen Weg der Bioaktivierung auszunutzen, der unabhängig von Cytochrom P450 (CYP450)-Enzymen ist. Dieses stellt ein unerwartetes Ergebnis dar, da bekannt ist, dass CYP450-Enzyme im Allgemeinen oxidative O-Desalkylierungen katalysieren, die im Falle des hier vorgeschlagenen Prodrug-Prinzips auch erforderlich wären, um den eigentlichen Arzneistoff freizusetzen.

Die vorgeschlagene Veretherung von N-O-haltigen Funktionalitäten mit Carboxyalkyl-Resten erbringt den besonderen Vorteil, dass ein anderes Enzym als die CYP450-Enzyme für eine Bioaktivierung ausgenutzt werden kann: die Peptidylglycin α-Amidierende Monooxygenase (PAM). Dadurch können beispielsweise Nebenwirkungen und die oben angesprochenen Interaktionen mit anderen gleichzeitig verabreichten Arzneistoffen vermieden werden.

Die Peptidylglycin α-Amidierende Monooxygenase (PAM) stellt in höheren Organismen (Wirbeltieren) ein bifunktionelles Enzym dar, welches aus einer Monooxygenase-Domäne (PHM, Peptidylglycin α-Hydroxylierende Monooxygenase, EC 1.14.17.3) und einer Lyase-Domäne (PAL, Peptidyl-α-hydroxyglycin α-Amidierende Lyase, EC 4.3.2.5) besteht. Insgesamt ist PAM einer stark gewebespezifischen und entwicklungsabhängigen Regulation durch *Splicing* und Expression unterworfen. Im Sinne einer posttranslationalen Modifikation vermag PAM diverse physiologisch vorkommende Peptidhormone, Neurotransmitter und Wachstumsfaktoren (z.B. Substanz P, Neuropeptid Y, Oxytocin, Vasopressin, Calcitonin) zu aktivieren. Dabei werden die Peptide C-terminal amidiert, indem ein endständiges Glycin mittels oxidativer *N*-Desalkylierung in einer Monooxygenase-Reaktion abgespalten wird.

Ein besonderer Vorteil der erfindungsgemäß vorgeschlagenen Veretherung der N-O-haltigen Funktionalitäten mit Carboxyalkyl-Resten ist die Verbesserung der Löslichkeit durch das Einführen einer unter physiologischen Bedingungen (pH 6-8) negativ geladenen Carbonsäure.

Ein weiterer Vorteil liegt darin, dass durch die erfindungsgemäß vorgeschlagene Veretherung der N-O-haltigen Funktionalitäten - unter Verwendung von (Alkoxycarbonyl)alkyl-Ethern bzw. (Aryloxycarbonyl)alkyl-Ethern - die Lipophilie soweit erhöht wird, dass eine passive Diffusion ermöglicht wird und damit die Bioverfügbarkeit und/oder Blut-Hirnschrankengängikeit verbessert wird.

Ebenfalls von Vorteil ist dabei die Möglichkeit, einen vergleichsweise kleinen Rest - im einfachsten Fall einen Carboxymethyl-Rest - als Prodrug-Gruppe zu verwenden, so dass die Größe des Arzneistoffmoleküls nur geringfügig steigt.

Wand et al. [Metabolism 1985, 34, 11, 1044] haben PAM-Aktivitäten in verschiedenen humanen Geweben untersucht und die höchste Aktivität in Geweben des ZNS (v.a. in der Hypophyse) detektiert. Keine Aktivität konnte hingegen in den klassischen Fremdstoffmetabolisierenden Organen Leber und Niere festgestellt werden. In Plasma, Herz und Lunge wurden ebenfalls Aktivitäten nachgewiesen, die für das angedachte Prodrug-Konzept ausgenutzt werden könnten.

Insbesondere die hohen Aktivitäten dieses Enzyms im ZNS kann ausgenutzt werden, um O-Carboxyalkylierte Prodrugs über die Blut-Hirnschranke zu transportieren, die dann umgewandelt werden können. Aber auch eine Bioaktivierung im kardiovaskulären System nach peroraler Applikation und Absorption aus dem Gastrointestinaltrakt ist möglich.

Das erfindungsgemäße Prodrug-System lässt sich dabei auf verschiedene Arzneistoffe anwenden, welche über eine Amidin oder Guanidin-Funktion verfügen. Besonders bevorzugt handelt es sich hierbei um die folgenden Arzneistoffe:
Pentamidin, Dabigatran, BSF 411693 (Abbott), Idazoxanhydrochlorid, Irbesartan, Linogliride, Lofexidinehydrochloride, Tetrahydrozolinhydrochlorid, Tolazolin, Xylometazolin-hydrochlorid, Pentamidinisethionat, Taribavirin, Thiamin (Vitamin B1), Bosentan, Dibrompropamidinisetionat, Hydroxystilbamidinisethionate, Sibrafiban, Orbofiban, Xemilofiban, Argatroban, Ximelagatran, Melagatran, 2-Piperidinsäure, Orbofibanazetat, Epinastin (Relestat), RO 43-8857, AB1 (Chlorambucil , Analoga), AMG-126737, AY-0068, B-623, BABIM, BIBT-986 (Boehringer Ingelheim), CI-1031 (Firma: Biosciences)), CJ-1332 (Finna: Curacyte), CJ-463 (Firma: Curacyte), CJ-672 (Firma: Curacyte), CT50728 (Portolla Pharmaceuticals), CVS-3983, DX-9065a, Lamifiban (Roche), LB-30870 (Firma: LG LifeSciences Ltd), LY-178550 (Firma: Lilly), PHA-927F und Analoga, RO-44-3888 (Roche), Sepimostat, FUT-187 (Torii), Viramidin (Ribapharm), WX-FX4 (Wilex), YM-60828 (Yamanouchi Pharmaceutical Co. Ltd), ZK-807191 (Berlex Biosciences), NAPAP (SR 25477), BIIL 315 (Boehringer Ingelheim), BIIL 260 (Boehringer Ingelheim), BIIL 284/260 (Boehringer Ingelheim), Tanogitran, Moxilubant, Stilbamidine, Panamidine, Fradafiban, Diminazene, Roxifiban, Furamidine, PD0313052, PHA 927F, PHA 798, Fidexabane, Otamixaban, Thromstop (Thrombstop), Zanamivir, Amiloridhydrochlorid, Anagrelidhydrochlorid, Proguanil, Cimetidin, Clonidinhydrochlorid, Guanoxa, Peramivir, Romifidine, Tirapazamine, Tizanidine, Tolo-nidinnitrat, Metformine, Diminazen, Debrisoquin, Sulfamethazine, Eptifibatide, Famotidine, Bayer-Arzneistoff, Streptomycin, Nafamostat, FUT-175, Inogatran, Guanethidin (Thilodigon), 3DP-10017, APC -366, CVS-1123, Diphenyphosphonat-derivat, E-64, FOY-305, MBGB, MIBG, RWJ-422521, Synthalin, WX-293, WX-340, BMS-189090, JTV-803, (Japan Tabacco), Napsagatran, Ismelin, Tan 1057A, Hydikal, Phenformix (Retardo), Netropsin (Sinanomycin), BIIB 722 (Sabiporide), Guanadrel, Deoxyspergualin, BMS 262084, Siamformet (Orabet), PPACK (Pebac), MERGETPA (Plummer's Carboxypeptidase Inhibitor), Peramivir, Famotidine, Zaltidine.

Im Anhang befindet sich eine Tabelle mit den Chemischen Formel, den CAS-Nummern und den Indikationen der Arzneistoffe.

Im folgenden sind exemplarisch 4 erfindungsgemäße Prodrugs gezeigt:

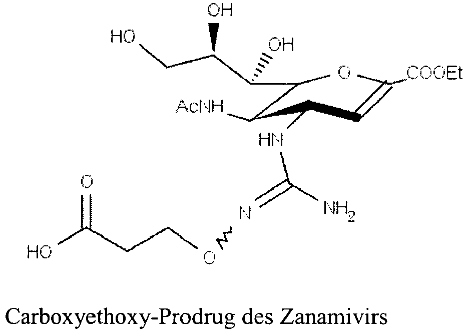

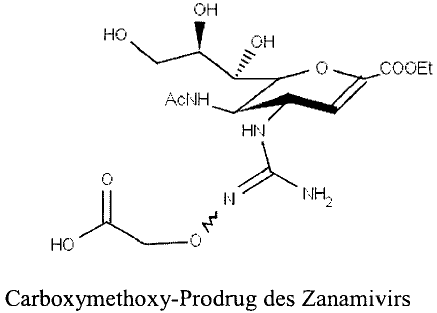

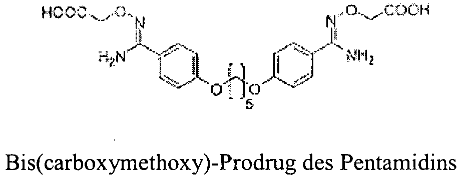

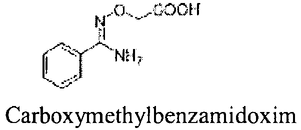

Die überaschende Entdeckung, dass auch nicht-peptidische *O*-carboxyalkylierte N-O-haltige Funktionalitäten als Substrate von PAM akzeptiert werden, wird in den Ausführungsbeispielen anhand von Amidoxim- und *N*-Hydroxyguanidin-basierten Modellverbindungen demonstriert.

Als Modellverbindung für Amidoxime wurde *O*-Carboxymethylbenzamidoxim (**1**) auf seine PAM-Substrateigenschaften getestet. *O*-Carboxymethylbenzamidoxim ist ein mögliches Prodrug des Arzneistoffs Benzamidin. Die PAM-katalysierte Bioaktivierung von *O*-Carboxymethylbenzamidoxim (**1**) zu Benzamidoxim (**2**) verläuft unter gleichzeitiger Freisetzung von Glyoxalsäure.

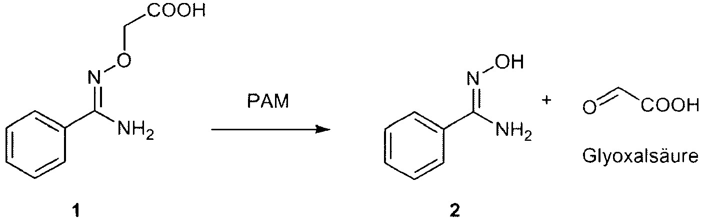

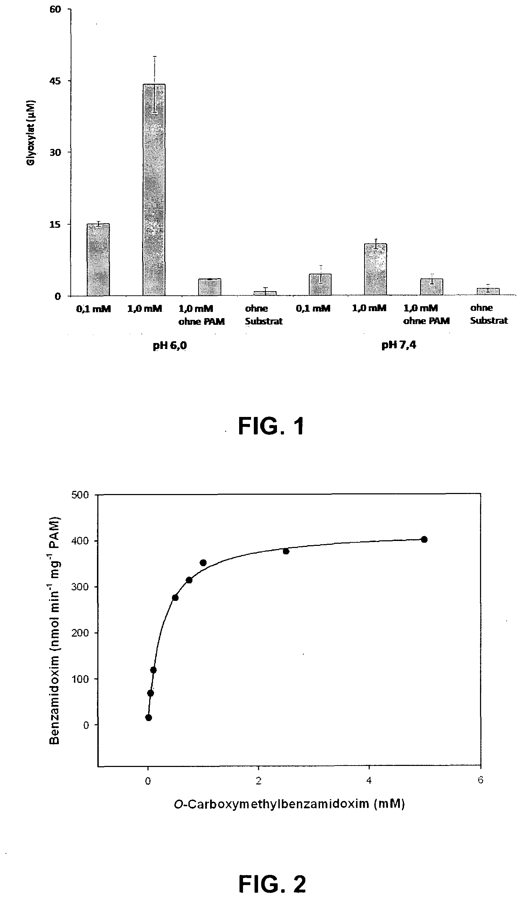

Abbildung 1 zeigt die Ergebnisse der kolorimetrischen Bestimmung der Glyoxalat-Bildung. Die ermittelten Glyoxylat-Konzentrationen sind Mittelwerte ± Standardabweichungen aus zwei Inkubationen, die jeweils doppelt vermessen wurden. Es kann die Bildung von Glyoxylat als Spaltprodukt der PAM-Katalyse von **1** in einer konzentrationsabhängigen Weise nachgewiesen werden. Die Inkubationen bei dem pH-Optimum von PAM (pH 6,0) resultierten, im Vergleich zur Inkubation bei pH 7,4, in deutlich höheren Umsetzungen. Im kolorimetrischen Assay erfolgte eine 5-Punkt-Kalibrierung von Glyoxylat parallel zur Testung von **1.** Die Kalibrierung war in dem ermittelten Konzentrationsbereich linear (r² = 1,000).

Da *O*-Carboxymethylbenzamidoxim (**1**) diesen Ergebnissen nach als Substrat von PAM akzeptiert wird, wurde die Reaktion durch Bestimmung der *K*_{M}- und *V*ₘₐₓ-Werte genauer charakterisiert.

Für diesen Zweck wurde eine HPLC-Analytik entwickelt. Die Kalibriergerade für Benzamidoxim war in dem ermittelten Konzentrationsbereich linear (r² = 1,000) und die Wiederfindungsrate betrug 130,6 % (r² = 0,999). Aus zwei unabhängigen Experimenten (n = 2) ergab sich ein *K*_{M}-Wert von 307 ± 80 µM und ein *V*ₘₐₓ-Wert von 393 ± 40 nmol min⁻¹ mg⁻¹ PAM. In Abbildung 2 ist eine solche Bestimmung repräsentativ dargestellt.

Für CYP450-Substratstudien wurde die oben angesprochene HPLC-Analytik so abgeändert, dass zusätzlich die Detektion des denkbaren Metaboliten Benzamidin, als Produkt aus der *N*-Reduktion von Benzamidoxim (**2**), möglich ist. Bei pH 6,0 sowie pH 7,4 konnten mit allen verwendeten CYP450-Enzymquellen weder Benzamidoxim (ein mögliches Prodrug des Benzamidins), noch Benzamidin nachgewiesen werden.

Auf Basis der Benzamidoxim-Modellverbindung **1** wird die *O*-Carboxymethyl-Funktion nur von PAM, nicht jedoch von Cytochrom P450 im Sinne einer Monooxygenase-Reaktion entfernt.

Als Modellverbindung für Hydroxyguanidine wurde *N*-Carboxymethoxy-*N',N"*-diphenylguanidin (**3**) auf seine PAM-Substrateigenschaften getestet.

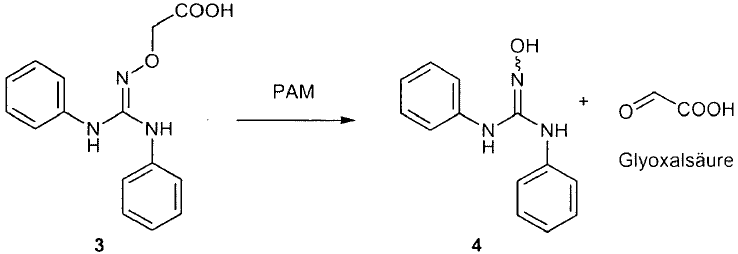

Die PAM-katalysierte Bioaktivierung von *N*-Carboxymethoxy-*N',N"*-diphenylguanidin (**3**) zu *N,N'*-Diphenyl-*N"*-hydroxyguanidin (**4**) verläuft unter gleichzeitiger Freisetzung von Glyoxalsäure.

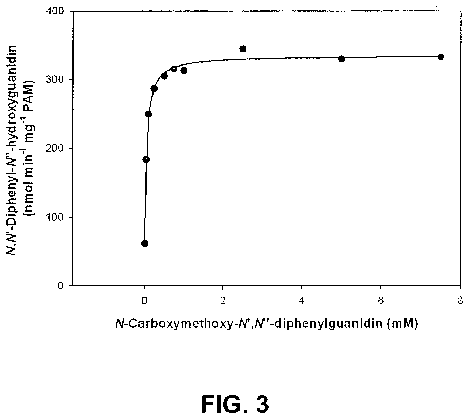

Die Ergebnisse im kolorimetrischen Assay mit **3** waren vergleichbar mit denen der Amidoxim-Modellverbindung **1.** Somit wurde zur Bestimmung der *K*_{M}- und *V*ₘₐₓ-Werte eine HPLC-Analytik entwickelt, die das Prodrug **3** und das Hydroxyguanidin **4** innerhalb von 15 Minuten auf einer RP-Säule zu trennen vermag. Die Kalibriergerade für *N,N*'-Diphenyl-*N*"-hydroxyguanidin (**4**) war in dem ermittelten Konzentrationsbereich linear (r² = 0,999) und die Wiederfindungsrate betrug 111,7 % (r² = 0,999). Aus zwei unabhängigen Experimenten (n = 2) ergab sich ein *K*_{M}-Wert von 37 ± 5 µM und ein *V*ₘₐₓ-Wert von 373 ± 53 µmol min⁻¹ mg⁻¹ PAM. In Abbildung 3 ist eine solche Bestimmung repräsentativ dargestellt.

Aus dem ermittelten *K*_{M}-Wert lässt sich eine ca. 8-fach höhere Affinität zu PAM im Vergleich zum Amidoxim-Prodrug **1** ableiten, während die Umsetzungsrate vergleichbar ist.

Für die CYP450-Substratstudien wurde die für die PAM-Substratstudien entwickelte HPLC-Analytik so abgeändert, dass zusätzlich die Detektion des denkbaren Metaboliten *N,N*'-Diphenylguanidin, als Produkt aus der *N*-Reduktion vom Hydroxyguanidin **4,** möglich ist. Bei pH 6,0 sowie pH 7,4 konnten mit allen verwendeten CYP450-Enzymquellen nach 180 Minuten Inkubationszeit weder **4,** noch *N,N*'-Diphenylguanidin nachgewiesen werden.

In Analogie zu *O*-Carboxymethylbenzamidoxim (**1**) wird auch auf Basis der Hydroxyguanidin-Modellverbindung **3** die *O*-Carboxymethyl-Funktion nur von PAM, nicht jedoch von Cytochrom P450 im Sinne einer Monooxygenase-Reaktion entfernt.

### Material und Methoden

### Natriumsalz des O-Carboxymethylbenzamidoxims Monohydrat (1)

Modifizierte Vorschrift nach Koch [*Ber. Dtsch. Chem. Ges.* 1889, 22, 3161]:

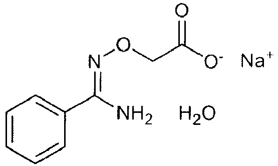

Eine Lösung von 681 mg Benzamidoxim (5,0 mmol), 1,04 g Bromessigsäure (7,5 mmol) und 500 mg Natronlauge-Plätzchen (12,5 mmol) in 5 mL Ethanol wird für 5 Stunden unter Rückfluss gekocht. Anschließend wird das Lösungsmittel so lange i. Vak. entfernt, bis sich ein Niederschlag anfängt zu bilden. Dieser wird zur vollständigen Fällung stehen gelassen, abfiltriert und getrocknet. Das Produkt wird aus Ethanol (96 %)/Wasser (95:5) umkristallisiert.
Ausbeute: 937 mg weiße, feinfilzige Kristalle (80 %)
Schmp.: 226 °C (dec.)
¹H-NMR (DMSO-*d*₆):
δ/ppm = 4.13 (s, 2H, O-CH₂), 6.09 (br s, 2H, NH₂), 7.37 (m, 3H, 3',4',5'-CH), 7.67 (m, 2H, 2',6'-CH).
¹³C-NMR (CDCl₃):
δ/ppm = 73.6 (O-CH₂), 125.7, 128.0, 129.0 (ArCH), 132.8 (ArC), 151.4 (C=N), 173.2 (CO).
MS (ESI):
m/z = 217 [M + Na]⁺, 195 [M + H]⁺, 119 [M - C₄H₂ - C₂H₂ +H]⁺, 105 [C₆H₅N₂]⁺.
C₉H₉N₂NaO₃·1,0 H₂O (234.18)
Ber. C 46.16 H 4.73 N 11.96
Gef. C 46.43 H 4.44 N 11.65

### N-Carboxymethoxy-N',N"-diphenylguanidin (3)

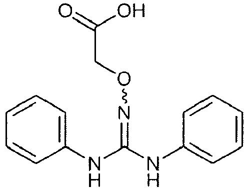

546 mg Aminooxyessigsäure Semichlorid (5 mmol) und 697 µl Triethylamin (5 mmol) werden für 30 min in 10 mL trockenem DMF gerührt. Das Präzipitat wird abfiltriert und zum Filtrat werden 970 mg *N,N*'-Diphenylcarbodiimid (5 mmol) zugegeben. Der Ansatz wird für vier Stunden bei Raumtemperatur gerührt, mit Ethylacetat ausgeschüttelt und das Produkt aus Ethanol umkristallisiert.
Ausbeute: 285 mg eines weißen Feststoffes (20 %)
Schmp.: 176 °C
DC: R_{f} = 0,29 (Dichlormethan/Methanol, 9:1)
¹H-NMR (DMSO-*d*₆):
δ/ppm = 4.37 (s, 2H, O-CH₂), 6.75-6.87 (m, 2H, ArH), 7.03-7.20 (m, 8H, ArH), 8.02, 8.21 (2 × br s, 1H, NH), 12.05 (br s, 1H, COOH).
¹³C-NMR (DMSO-*d*₆):
δ/ppm = 70.0 (O-CH₂), 116.7, 118.7, 119.8, 121.0, 128.5 (ArCH), 140.7, 142.3 (ArC), 147.5 (C=N), 171.8 (CO).
MS (ESI):
m/z = 308 [M + Na]⁺, 286 [M + H]⁺, 210 [M - C₂H₄O₃]⁺.
MS (EI):
m/z (%) = 209 (38), 208 (37), 119 (20), 118 (38), 93 (100), 91 (47), 77 (43), 66 (31), 51 (30).
C₁₅H₁₅N₃O₃·0,3 H₂O (290.71)
Ber. C 61.97 H 5.41 N 14.45
Gef. C 62.18 H 5.72 N 14.57

### HPLC-System:

Waters Breeze HPLC-System mit Waters 1525 Pumpen, Waters 2487 Absorptions-detektor, Waters 717 Plus Autosampler und Breeze Aufnahme- und Auswertesoftware (Version 3.30), Gynkotek STH 585 Säulenofen.

### HPLC-Säulen:

Synergi Max-RP 80 A (250 × 4.6 mm, 4 µm) mit Vorsäule C-18 (4 × 3 mm) (Fa. Phenomenex);
LiChroCART, LiChrospher 100, RP-8 (125 × 4 mm, 5 µm) mit Vorsäule LiChrospher 60, RP-select B (4 × 4 mm, 5 µm) (Fa. Merck);
LiChroCART, LiChrospher RP-select B (250 × 4.6 mm, 5 µm) mit Vorsäule LiChrospher 60, RP-select B (4 × 4 mm, 5 µm) (Fa. Merck).

### Weitere Geräte und Materialien:

Cary 50 UV-Vis Photometer (Fa. Varian); 96-well Platten (Fa. Greiner); Schüttelwasserbad GFL-1083 (Gesellschaft für Labortechnik, Burgwedel); Mikroliter-Zentrifuge (Fa. Hettich GmbH); pH-Messgerät InoLab pH Level 1 (Wissenschaftlich-Technische Werkstätten GmbH, Weilheim) mit einer pH-Elektrode LiQ Plast (Fa. Hamilton); Vortexer VF2 (Janke und Kunkel GmbH & Co. KG, Staufen); Reaktionsgefäße 1,5 mL (Sarstedt AG & Co., Nümbrecht).

### Enzymquellen:

Die verwendete rekombinante Peptidylglycin-α-Amidierende Monooxygenase (PAM, Ratte, EC 1.14.17.3) wurde von der Firma Unigene Laboratories, Inc. (New Jersey, USA) zur Verfügung gestellt (spezifische Aktivität = 5,8 10⁶ U/mg Protein); Bovine Leber-Katalase (EC 1.11.1.6), spezifische Aktivität = 12600 U/mg Feststoff (Fa. Aldrich).

Die Gewinnung der verwendeten Cytochrom P450-Enzymquellen erfolgte im Arbeitskreis Clement von Grünewald nach folgenden Vorschriften:
Porcine Lebermikrosomen und 9000 g Überstände:
   Die Schweinelebern wurden von einem lokalen Schlachter (Bordesholm) bezogen und die Organe direkt nach der Schlachtung in einem eisgekühlten 20 mM Phosphatpuffer (1 mM Na₂-EDTA, pH 7,4) transportiert. Zur Weiterverarbeitung wurden die Leberlappen zunächst mit 50 mM Phosphatpuffer (1 mM Na₂-EDTA, pH 7,4) perfundiert und gewaschen. Das Gewebe wurde in Stücke geschnitten und durch einen handelsüblichen Fleischwolf gegeben. Die Suspension wurde mit dem gleichen Volumen Phosphatpuffer verdünnt und mit einem Durchflusshomogenisator homogenisiert. Die weitere Gewinnung der Mikrosomen und 9000 g Überstände erfolgte durch differentielle Ultrazentrifugation. Zur Lagerung wurden die erhaltenen Präparationen aliquotiert und bei -80 °C eingefroren.
Humane Lebermikrosomen und 9000 *g* Überstände:
   Zur Gewinnung humaner Mikrosomen wurde humanes Lebergewebe von Karzinom-Patienten der Chirurgie des Uni-Klinikums der Christian-Albrechts-Universität erhalten, die sich einer Hemihepatektomie unterziehen mussten.

Die Lebergewebestücke wurden in einem Saccharose-haltigen Phosphatpuffer (10 mM K2HPO4, 10 mM KH2PO4, 250 mM Saccharose, 1 mM Na2-EDTA, pH 7,4, 4 °C) schockgefroren. Sobald eine ausreichende Anzahl an Organteilen (> 3) vorhanden war, wurden die entsprechenden Stücke aufgetaut und gepoolt, um Unterschiede durch interindividuelle Schwankungen auszugleichen. Die Gewebestücke wurden bei 4 °C in kleinere Teile geschnitten, mehrfach mit Puffer-Lösung (ohne EDTA) gewaschen und mit einem Homogenisator zu einer Suspension verarbeitet. Die Gewinnung der Mikrosomen und 9000 g Überstände erfolgte aus dieser Suspension durch differentielle Ultrazentrifugation. Zur Lagerung wurden die erhaltenen Präparationen aliquotiert und bei -80 °C eingefroren.

### PAM-Assay: Inkubationsbedingungen

Ein typischer Inkubationsansatz von 300 µL (Totalvolumen) enthielt 25000 U/mL Peptidylglycin-α-Amidierende Monooxygenase (PAM, Firma: Unigene Laboratories), 250 U/mL Katalase, 1 µM Kupfer(II) (eingesetzt als Acetat/Monohydrat), 2 mM Natriumascorbat, 5 mM Kaliumiodid und das jeweilige Substrat in 0,1 mM oder 1 mM Konzentration, in Puffer unterschiedlichen pH-Werts. Als Puffersystem wurden 30 mM MES für die Inkubation bei pH 6,0 und 50 mM HEPES für die Inkubation bei pH 7,4 verwendet. Der pH-Wert wurde jeweils mit verdünnter Natronlauge eingestellt. Es wurde bei 37 °C im Schüttelwasserbad 60 min inkubiert, 100 µL abgenommen und die Reaktion mit 50 µL 10 %-iger TFA_{(aq)}/Acetonitril (2:3) abgestoppt. Der restliche Ansatz wurde weitere 180 min bei 37 °C inkubiert und mit 100 µL 10 %-iger TFA_{(aq)}/Acetonitril (2:3) abgestoppt.

Die abgestoppten Proben wurden 5 min geschüttelt (Vortexer) und bei -80 °C eingefroren. Zur Analyse der Proben wurden diese aufgetaut, 5 min geschüttelt und das gefällte Protein bei 10000 U/min zentrifugiert. Der Überstand wurde für die kolorimetrische Glyoxylat-Bestimmung und/oder HPLC-Vermessung verwendet.

Für die *K*_{M}- und *V*ₘₐₓ-Bestimmung wurden 100 µL-Ansätze bei pH 6,0 unter den oben beschriebenen Bedingungen durchgeführt, jedoch mit dem Unterschied einer Inkubationszeit von 30 min.

### Kolorimetrische Bestimmung von Glyoxylat

200 µL des vom Protein befreiten Inkubationsansatzes wurden mit 20 µL einer Phenylhydrazin-Lösung (20 mg in 2 mL *Aqua bidest*.) versetzt und 5 min im Schüttelwasserbad bei 37 °C geschüttelt. Anschließend wurde für 15 min auf 0 °C gekühlt, 100 µL eiskalte 6 N HCl zugegeben und weitere 5 min bei 0 °C stehen gelassen. Abschließend erfolgte die Zugabe von 20 µL einer Kaliumhexacyanoferrat(III)-Lösung (100 mg in 2 mL *Aqua bidest*.). Der Ansatz wurde für 15 min bei Raumtemperatur stehen gelassen und 200 µL für die Vermessung mit einem Plattenreader (Cary 50 UV-Vis Photometer, 520 nm) entnommen.
Kalibrierung:
Für eine 5-Punkt-Kalibrierung wurde Glyoxalsäure in Konzentrationen von 2, 5, 10, 50 und 100 µM in einem 2:1-Gemisch von Assay-Puffer (pH 6,0):10 %-iger TFA_{(aq)}/Acetonitril (2:3) wie oben beschrieben vermessen. Diese Kalibrierung erfolgte parallel bei jedem durchgeführten Assay einer Testverbindung.

### HPLC-Analytik zur Trennung von O-Carboxymethylbenzamidoxim (1) und Benzamidoxim (2)

| | |
|---|---|
| Säule: | Synergi Max-RP 80 A (250 × 4.6 mm, 4 µm) |
| Säulentemperatur: | 20 °C |
| Mobile Phase: | 79 % (v/v) 10 mM Octylsulfonat, pH 2,5 (H₃PO₄) |
| | 21 % (v/v) Acetonitril |
| Flussrate : | 1,0 mL/min |
| Laufzeit: | 20 min |
| Detektion: | Absorptionsmessung bei 229 nm |
| Injektionsvolumen: | 20 µL |
| Retentionszeiten: | |
| *O*-Carboxymethylbenzamidoxim **(1)** | 8,9 min ± 0,2 min |
| Benzamidoxim (**2**) | 14,4 min ± 0,2 min |

### Kalibrierung und Wiederfindung:

Zur Kalibrierung wurde Benzamidoxim in acht Konzentrationen von 0,1-500 µM, gelöst in Assay-Puffer (30 mM MES, 1 µM Kupfer(II)-acetat, 2 mM Natriumascorbat, 5 mM Kaliumiodid, pH 6,0), mit der oben beschriebenen HPLC-Methode vermessen.

Für die Bestimmung der Wiederfindung wurden die gleichen Konzentrationen in Assay-Puffer hergestellt (Endvolumen = 100 µL). Weiterhin wurde O-Carboxymethylbenzamidoxim (0,5 mM) und 250 U/mL Katalase zugegeben sowie abschließend 50 µL 10 %-iger TFA_{(aq)}/Acetonitril (2:3). Die Proben wurden mit dem Vortexer geschüttelt und bei -80 °C eingefroren. Zur Vermessung wurden die Proben aufgetaut, 5 min am Vortexer geschüttelt und 5 min bei 10000 U/min zentrifugiert.

### HPLC-Analytik zur Trennung von N-Carboxymethoxy-N',N"-diphenylguanidin (3) und N-Hydroxy-N',N"-diphenylguanidin (4)

| | |
|---|---|
| Säule: | LiChrospher RP-select B (250 × 4.6 mm, 5 µm) |
| Säulentemperatur: | 20 °C |
| Mobile Phase: | 70 % (v/v) 40 mM Ammoniumacetat, pH 5,2 |
| | 30 % (v/v) Acetonitril |
| Flussrate : | 1,0 mL/min |
| Laufzeit: | 15 min |
| Detektion: | Absorptionsmessung bei 229 nm |
| Injektionsvolumen: | 20 µL |

Retentionszeiten:

| | |
|---|---|
| *N*-Carboxymethoxy-*N*'*,N*"-diphenylguanidin (**3**) | 5,2 min ± 0,1 min |
| *N*-Hydroxy-*N*',*N*"-diphenylguanidin (**4**) | 9,0 min ± 0,2 min |

### Kalibrierung und Wiederfindung:

Zur Kalibrierung wurde *N*-Hydroxy-*N*',*N*"-diphenylguanidin (**4**) in acht Konzentrationen von 0,1-500 µM, gelöst in Assay-Puffer (30 mM MES, 1 µM Kupfer(II)acetat, 2 mM Natriumascorbat, 5 mM Kaliumiodid, pH 6,0), mit der oben beschriebenen HPLC-Methode vermessen.

Für die Bestimmung der Wiederfindung wurden die gleichen Konzentrationen in Assay-Puffer hergestellt (Endvolumen = 100 µL). Weiterhin wurde *N*-Carboxymethoxy-*N*',*N*"-diphenylguanidin (**3**) (0,5 mM) und 250 U/mL Katalase zugegeben sowie abschließend 50 µL 10 %-iger TFA_{(aq)}/Acetonitril (2:3). Die Proben wurden mit dem Vortexer geschüttelt und bei -80 °C eingefroren. Zur Vermessung wurden die Proben aufgetaut, 5 min am Vortexer geschüttelt und 5 min bei 10000 U/min zentrifugiert.

### CYP450-Assay: Inkubationsbedingungen

Ein typischer Inkubationsansatz von 500 µL (Totalvolumen) enthielt 0,3 mg Protein (Schweine- oder Humanleberenzymquelle), 0,1 mM (oder 1 mM) Testverbindung in 100 mM Phosphatpuffer (pH 6,0 oder pH 7,4) und 1 mM NADH (oder NADPH). Die Inkubation wurde nach einer 5-minütigen Vorinkubation von Enzym und Testverbindung in Puffer durch den Zusatz von NADH (bzw. NADPH) gestartet und 60 min bzw. 180 min bei 37 °C im Schüttelwasserbad geschüttelt. Die Ansätze wurden durch Zusatz des gleichen Volumens Acetonitril gestoppt, auf dem Vortexer geschüttelt und bei -80 °C eingefroren.

Zur Analyse wurden die Proben aufgetaut, 5 min auf dem Vortexer geschüttelt und das Protein durch 5-minütige Zentrifugation bei 10000 U/min abgetrennt. Der Überstand wurde für die HPLC-Analytik verwendet.

### HPLC-Analytik zur Trennung von O-Carboxymethylbenzamidoxim (1), Benzamidoxim (2) und Benzamidin

| | |
|---|---|
| Säule: | Synergi Max-RP 80 A (250 × 4.6 mm, 4 µm) |
| Säulentemperatur: | 20 °C |
| Mobile Phase: | 82,5 % (v/v) 10 mM Octylsulfonat, pH 2,5 (H₃PO₄) |
| | 17,5 % (v/v) Acetonitril |
| Flussrate : | 1,0 mL/min |
| Laufzeit: | 35 min |
| Detektion: | Absorptionsmessung bei 229 nm |
| Injektionsvolumen: | 20 µL |
| Retentionszeiten: | |
| *O*-Carboxymethylbenzamidoxim (**1**) | 13,6 min ± 0,1 min |
| Benzamidoxim (**2**) | 22,8 min ± 0,3 min |
| Benzamidin | 26,0 min ± 0,3 min |

### HPLC-Analytik zur Trennung von N-Carboxymethoxy-N',N"-diphenylguanidin (3), N-Hydroxy-N',N"-diphenylguanidin (4) und N,N'-Diphenylguanidin

| | |
|---|---|
| Säule: | LiChrospher RP-select B (250 × 4.6 mm, 5 µm) |
| Säulentemperatur: | 20 °C |
| Mobile Phase: | 80 % 20 mM Ammoniumacetat, pH 4,3 |
| | 20 % Acetonitril |
| Flussrate : | 1,25 mL/min |
| Laufzeit: | 15 min |
| Detektion: | Absorptionsmessung bei 205 nm |
| Injektionsvolumen: | 30 µL |
| Retentionszeiten: | |
| *N,N'*-Diphenylguanidin | 6,7 min ± 0,2 min |
| *N*-Carboxymethoxy-*N*',*N*"-diphenylguanidin (**3**) | 7,8 min ± 0,2 min |
| *N*-Hydroxy-*N*',*N*"-diphenylguanidin (**4**) | 10,7 min ± 0,3 min |

Es folgt eine Tabelle der Arzneistoffe, auf die das erfindungsgemäße Prodrug-System bevorzugt angewendet werden kann:

| **Struktur** | **Substanzname** | CAS | **Wirkung/ Indikation** |
|---|---|---|---|
| 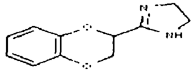 | **Idazoxan- Hydrochlorid** | 79944-58-4 | selektiver α2 adrenerger Rezeptor Antagonist und Antagonist des Imidazolinrezeptors. Zunächst als Antidepressivum getestet, jetzt gegen Schizophrenie in Untersuchung |
| 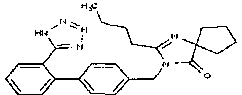 | **Irbesartan** | 138402-11-6 | Angiotensin-II-Rezeptor-Antagonist wie die meisten Sartane gegen Bluthochdruck |
| 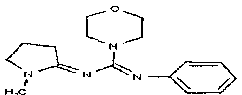 | **Linogliride** | 75358-37-1 | gegen Hyperglykämie |
| 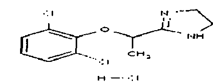 | **Lofexidine hydrochloride** | 21498-08-8 | α2 adrenerger Rezeptor Antagonist, früher als Blutdrucksenker, heute hauptsächlich gegen Heroin- und Opiat Entzugserscheinungen |
| 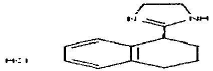 | **Tetrahydrozoline hydrochloride** | 522-48-5 | in Augentropfen und Nasenspray, alpha-Antagonist |
| 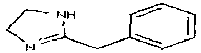 | **Tolazolin** | 59-98-3 | nicht selektiver, kompetitiver α2 adrenerger Rezeptor Antagonist. Wirkt gefäßerweitemd, meist in der Veterinärmedizin als Aufweckmittel genutzt |
| 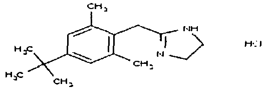 | **Xylometazolin Hydrochlorid** | 1218-35-5 | Nasenspray gegen Schnupfen etc. |
| 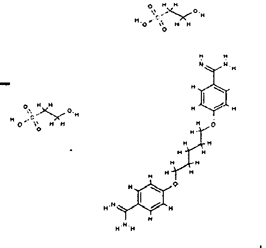 | **Pentamidin isethionat** | 140-64-7 | anti-infektiös Antiprotozoal agent effective in trypanosomiasis, leishmaniasis, and some fungal infections |
| | | | |
| 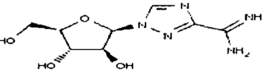 | **Taribavirin** | 119567-79-2 | Polymerasehemmer gegen Hepatitis C |
| 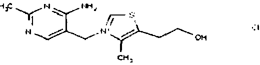 | **Thiamin (Vitamin B1)** | 59-43-8 | Vitamin-B1-Mangel |
| 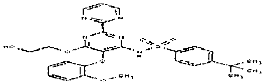 | **Bosentan** | 147536-97-8 | Endothelin-Rezeptorantagonist zur Behandlung pulmonaler arterieller Hypertonie |
| 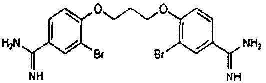 | **Dibrompropamidine isetionate** | 496-00-4 | Antiseptikum, Augentropfen |
| 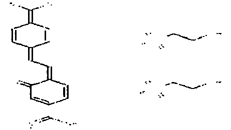 | **Hydroxystilbamidine Isethionate** | 495-99-8 | treating various fungal infections |
| 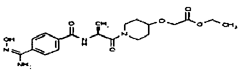 | **Sibrafiban** | 172927-65-0 | GPIIb/IIIa Inhibitor |
| 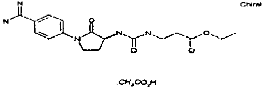 | **Orbofiban** | 163250-90-6 | Antiplatelet drug |
| 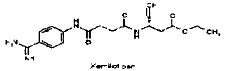 | **Xemilofiban** | 149820-74-6 | Glykoprotein IIb/IIa Antagonist als Gerinnungshemmer |
| 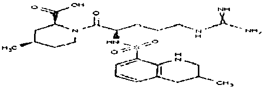 | **Argatroban** | 74863-84-6 | Antikoagulantien |
| | | 141396-28-3 | |
| 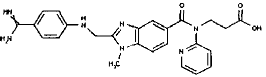 | **Dabigatran** | 211914-51-1 | Antikoagulantien |
| 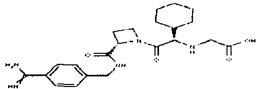 | **Ximelagatran/ Melagatran** | 159776-70-2 | Thrombinhemmer |
| 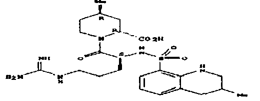 | **2-Piperidinecarboxylic acid** | 74863-84-6 | Thrombinhemmer |
| 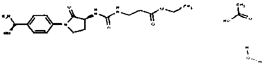 | **Orbofiban acetate** | 165800-05-5 | Glykoprotein IIb/IIIa-rezeptor Antagonist |
| 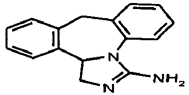 | **Epinastin (Relestat)** | 127786-29-2 | Antihistaminikum |
| 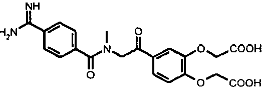 | **RO 43-8857** | 1322224-71-6 | GPIIB/IIIa Antagonist, Antikoagulant |
| 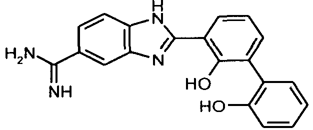 | | | FVIIa Inhibitor, Antikoagulant, Präklinik |
| 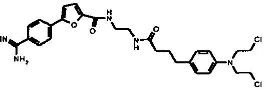 | **AB₁ (Chlorambucil Analoga)** | 305-03-3 | Leukämie, Lymphome, Brustkrebs, Präklinik |
| 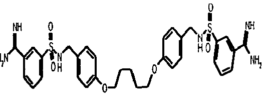 | **AMG-126737** | 224054-76-6 | Tryptase Inhibitor, Präklinik |
| 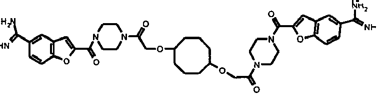 | **AY-0068** | | Tryptase Inhibitor, Präklinik |
| 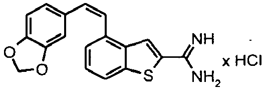 | **B-623** | | Urokinase Inhibitor, maligne Erkrankungen, Präklinik |
| 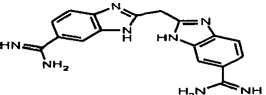 | **BABIM** | 74733-75-8 | Tryptase Inhibitor, Allergie, Asthma. Präklinik |
| 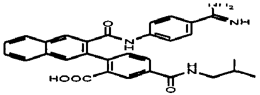 | | 1670-14-0 | Faktor VIIa Inhibitor, Antikoagulant. Präklinik |
| 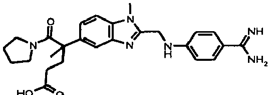 | **BIBT-986 (Tanogitran) (Boehringer Ingelheim)** | 637328-69-9 | Duale FXa und Thrombin Inhibitoren, Antikoagulant |
| 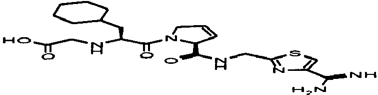 | **BSF 411693 (Abbott)** | | Thrombin Inhibitor, Antikoagulant, Präklinik |
| 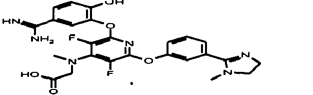 | **CI-1031 (Firma: Biosciences)** | 605-69-6 | FXa Inhibitor, Antikoagulant. Phase II |
| 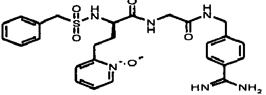 | **CJ-1332 (Firma: Curacyte)** | | FXa Inhibitor, Antikoagulant, Präklinik |
| 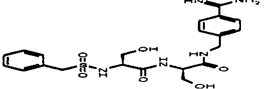 | **CJ-463 (Firma: Curacyte)** | | Urokinase Inhibitor, maligne Erkrankungen, Präklinik |
| 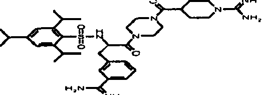 | **CJ-672 (Firma: Curacyte)** | | Matriptase Inhibitor, Therapie maligner Erkrankungen, Präklinik |
| 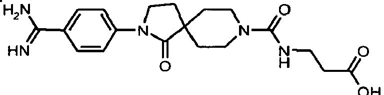 | **CT50728 (Portolla Pharmaceuticals)** | | Glykoprotein IIb/IIa Antagonist als Gerinnungshemmer |
| 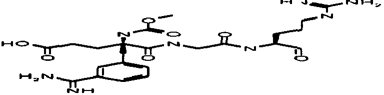 | **CVS-3983** | | Matriptase Inhibitor, maligne Erkrankungen, Präklinik |
| 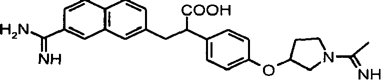 | **DX-9065a** | 155204-81-2 | FXa Inhibitor, Antikoagulant. Phase III |
| 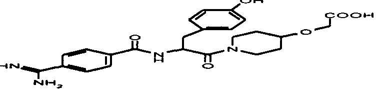 | **Lamifiban (Roche)** | 103577-45-3 | Glykoprotein IIb/IIa Antagonist als Gerinnungshemmer |
| 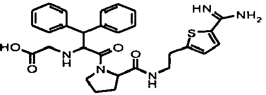 | **LB-30870 (Firma: LG LifeSciences Ltd)** | | Thrombin Inhibitor, Antikoagulant, Phase I |
| 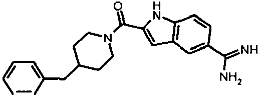 | **LY-178550 (Firma: Lilly)** | | Thrombin Inhibitor, Antikoagulant. Präklinik |
| 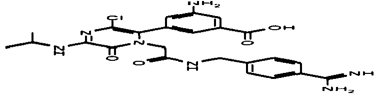 | **PHA-927F und Analoga** | | Faktor Va Inhibitor, Antikoagulant |
| 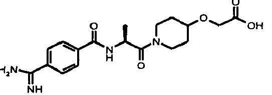 | **RO-44-3888 (Roche)** | | Glykoprotein IIb/IIa Antagonist als Gerinnungshemmer |
| 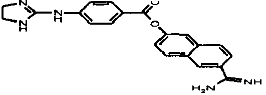 | **Sepimostat, FUT-187 (Torii)** | 103926-64-3 | C3/C5 Convertase Inhibitor, Komplement Aktivierung, Antiphlogistikum, Phase II |
| 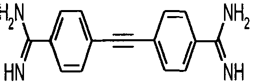 | **Stilbamidin (analog Diminazen, Berenil)** | 140-59-0 | Trypanosomiasis |
| 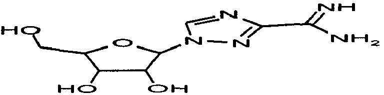 | **Viramidin (Ribapharm)** | 119567-79-2 | Virustatikum (Hepatitis C), Phase III |
| 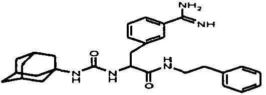 | **WX-FX4 (Wilex)** | | FXa Inhibitor. Antikoagulant. Präklinik |
| 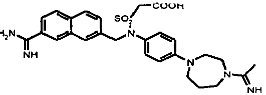 | **YM-60828 (Yamanouchi Pharmaceutical Co. Ltd)** | | FXa Inhibitor, Antikoagulant, Präklinik |
| 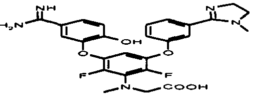 | **ZK-807191 (Berlex Biosciences)** | | FXa Inhibitor, klinische Prüfung |
| 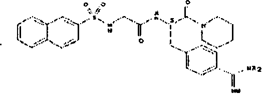 | **NAPAP, SR25477** | 86845-59-2 | Analgetikum |
| 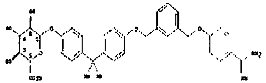 | **BIIL 315 (Boehringer Ingelheim)** | 204974-94-7 | Entzündungshemmer |
| 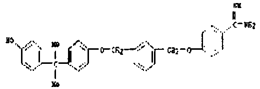 | **BIIL 284/260 (Boehringer Ingelheim)** | 204974-93-6 | Entzündungshemmer |
| 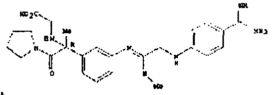 | **Tanogitran** | 637328-69-9 | Thrombinhemmer |
| 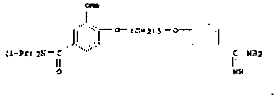 | **Moxilubant** | 146978-48-5 | Antiasthmatikum, Antiinfektivum |
| 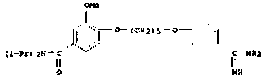 | **Stilbamidin** | 122-06-5 | Antiprotozoikum, Antiinfektivum, Fungistatikum |
| 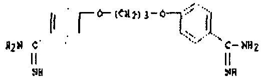 | **Panamidin** | 104-32-5 | Antiprotozoikum, Antiinfektivum |
| 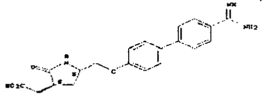 | **Fradafiban** | 148396-36-5 | Thrombinhemmer |
| 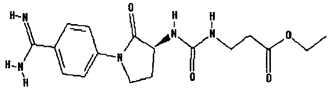 | **Orbofiban** | 163250-90-6 | Glykoprotein IIb/IIa Antagonist als Gerinnungshemmer |
| 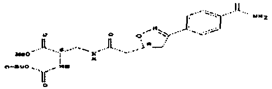 | **Roxifiban** | 170902-47-3 | Glykoprotein IIb/IIa Antagonist als Gerinnungshemmer |
| 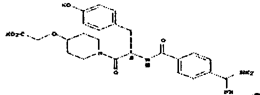 | **Lamifiban (Roche)** | 144412-49-7 | Thrombinhemmer |
| 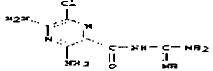 | **Furamidine** | 73819-26-8 | Antiprotozoikum, Antiinfektivum |
| 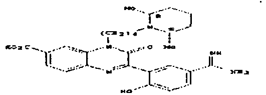 | **PD0313052** | 861244-44-2 | Thrombinhemmer |
| 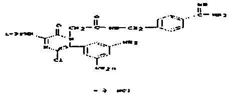 | **PHA 927F** | 648943-12-8 | spezifischer TF/VIIa Hemmstoff |
| 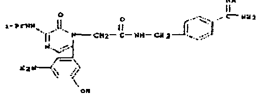 | **PHA 798** | 508173-28-2 | spezifischer TF/VIIa Hemmstoff |
| 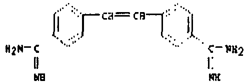 | **Stilbamidine** | 122-06-5 | Antiprotozoikum, Antiinfektivum |
| 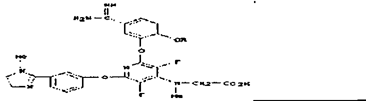 | **Fidexaban (ZK807834)** | 183305-24-0 | Antikoagulant |
| 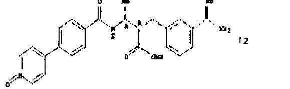 | **Otamixaban** | 193153-04-7 | Faktor Xa-Inhibitor |
| 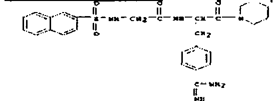 | **Thrombstop** | 117091-16-4 | Thrombinhemmer |
| 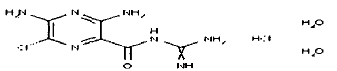 | **Amilorid Hydrochlorid** | 2016-88-8 | Diuretikum |
| 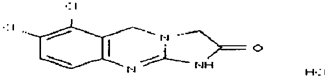 | **Anagrelid hydrochlorid** | 58579-51-4 | Reduziert Blutplättchen |
| 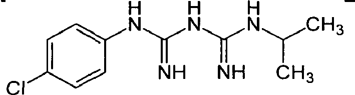 | **Proguanil** | 537-21-3 | Prophylaktisches Anitmalariamittel (gegen Plasmodium) |
| 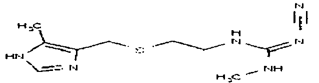 | **Cimetidin** | 51481-61-9 | H2-Rezeptor-Antagonist (gegen Sodbrennen) |
| 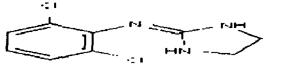 | **Clonidin Hydrochlorid** | 4205-91-8 | direkter α2 adrenerger Agonist Behandlung der Hypertonie * Behandlung von Drogenentzugssyndromen (Alkohol. Opioide u. a.) * Bei Alkohol keine Monotherapie |
| 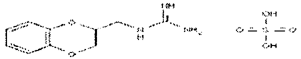 | **Guanoxan** | 19694-60-1 | Blutdrucksenker |
| 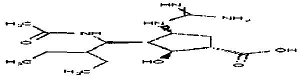 | **Peramivir** | 229614-55-5 | Neuramidase-Hemmer (Influenza), zugelassen für den Notfall bei H1N1-Infektionen, intraevenös |
| 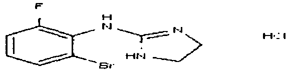 | **Romifidine** | 65896-14-2 | α2 adrenerger Rezeptor Agonist, Veterinärmedizin: Sedativum, Anästhetikum, Analgetikum für große Tiere wie Pferde |
| 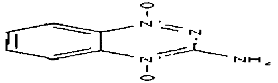 | **Tirapazamine** | 27314-97-2 | Experimenteller Anti-Tumorwirkstoff. setzt kleine Mengen toxischer Radikale frei. Leitstruktur für weitere Krebsmedikamente |
| 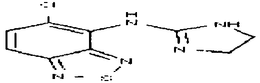 | **Tizanidine** | 51322-75-9 | α2 adrenerger Rezeptor Agonist, Muskelrelaxans gegen Spastiken, Krämpfe etc. |
| 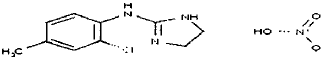 | **Tolonidin nitrat** | 57524-15-9 | |
| 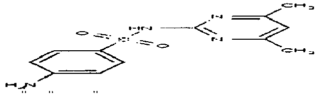 | **Metformin** | 657-24-9 | Diabetes mellitus Typ 2 |
| 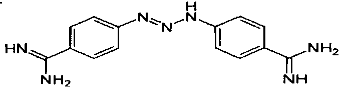 | **Diminazene** | 536-71-0 | Antiprotozoikum, Antiinfektivum |
| 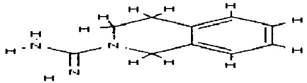 | **Debrisoquin** | 1131-64-2 | Antihypertensivum |
| 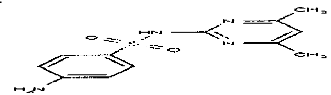 | **Sulfamethazine** | 57-68-1 | Zusatz in Tiemahrung |
| 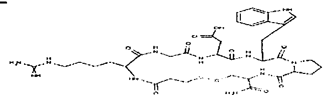 | **Eptifibätide** | 188627-80-7 148031-34-9 | Antikoagulant |
| 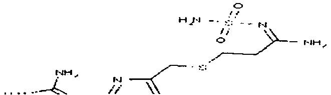 | **Famotidine** | 76824-35-6 | Na+ H+-Transportinhibitor |
| 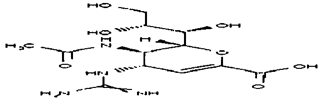 | **Zanamivir** | 139110-80-8 | Neuramidase-Hemmer (Influenza) |
| 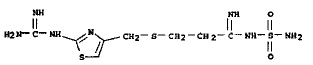 | **Bayer-Arzneistoff** | **25836-74-2** | |
| 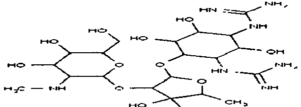 | **Streptomycin A** | 57-92-1 | Antibiotikum |
| 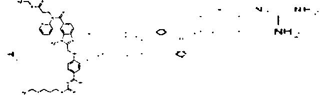 | **Nafamostat** | 81525-10-2 | |
| 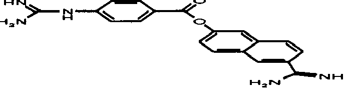 | **Nafamostat, FUT-175** | 81525-10-2 | C3/C5 Convertase Inhibitor, Komplement, Aktivierung. Antiphlogistikum, Phase II |
| 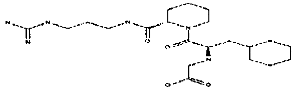 | **Inogatran** | 155415-08-0 | |
| 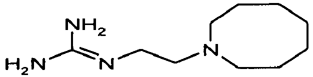 | **Guanethidin (Thilodigon)** | 645-43-2 | Hypertonikum, Glaukom |
| 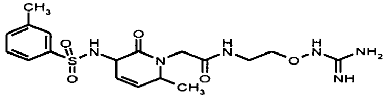 | **3DP-10017** | 226566-43-4 | Dualer FXa und Thrombin Inhibitor, Kinische Entwicklung |
| 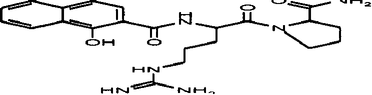 | **APC-366** | 178925-65-0 | Tryptase Inhibitor, Allergie, Asthma, Phase II |
| 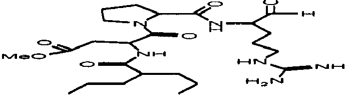 | **CVS-1123** | | Thrombin Inhibitor, klinische Prüfung |
| 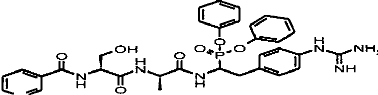 | **Diphenyphosphonatderivate** | 5945-33-5 | Urokinase Inhibitor, maligne Erkrankungen, Präklinik |
| 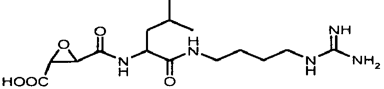 | **E-64** | 66701-25-5 | Cathepsin L Inhibitor, maligne Erkrankungen. Präklinik |
| 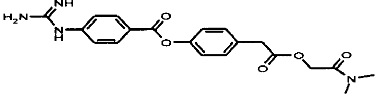 | **FOY-305** | 59721-28-7 | Breitspektrum Serin Protease Inhibitoren (Pankreatitis, Antikoagulanz), klin, Entwicklung |
| 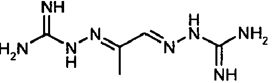 | **MBGB** | | Lymphome, Leukämie, Klinik |
| 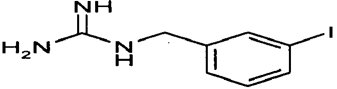 | **MIBG** | 103346-16-3 | Neuroblastom, Klinik |
| 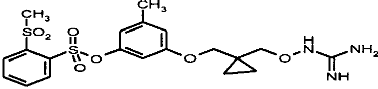 | **RWJ-422521** | | Dualer FXa und Thrombin Inhibitor, Kinische Entwicklung |
| 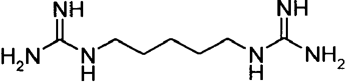 | **Synthalin** | 301-15-5 | Antidiabetikum |
| 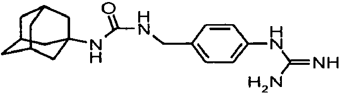 | **WX-293** | | Urokinase Inhibitor, maligne Erkrankungen, Präklinik |
| 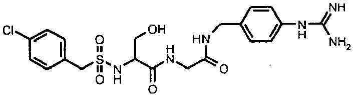 | **WX-340** | | Amyotropische Lateralsklerose, Malignome, Phase I |
| 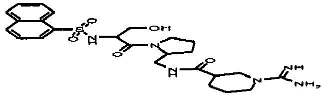 | **BMS-189090** | | Thrombin Inhibitor klinische Prüfung |
| 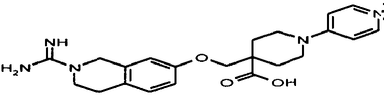 | **JTV-803 (Japan Tabacco)** | | FXa Inhibitor, Antikoagulant. Phase II |
| 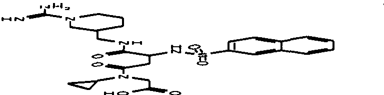 | **Napsagatran** | 159668-20-9 | Thrombin Inhibitor, Antikoagulant, klinische Entwicklung |
| 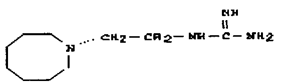 | **Abapressin, Azocine, Dopom,** | 55-65-2 | Hypertonikum |
| 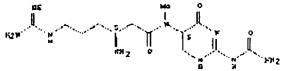 | **TAN 1057A** | 128126-44-3 | Antibiotikum |
| 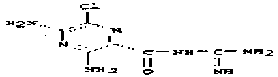 | **Hydikal, MK 870** | 2609-46-3 | Herz-Rhythmus-Stöung |
| 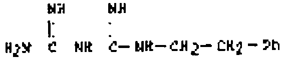 | **Phenformin, Retardo** | 114-86-3 | Diabetes Mellitus II |
| 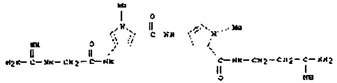 | **Netropsin, Sinanomycin** | 1438-30-8 | Antibiotikum |
| 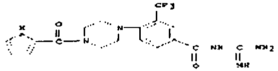 | **BIIB, Sabiporide** | 261505-80-0 | Na+ H+-Transportinhibitor |
| 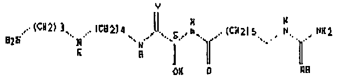 | **Deoxyspergualin** | 89149-10-0 | Immunsuppressivum |
| 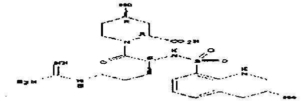 | | | |
| 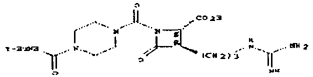 | **BMS 262084** | 253174-92-4 | Tryptase Inhibitor |
| 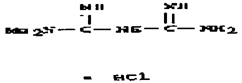 | **Apophage, Siamformet** | 1115-70-4 | Diabetes Mellitus II |
| 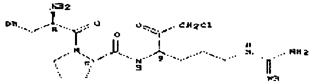 | **Pebac, L-Prolinamide** | 71142-71-7 | Thrombinhemmer |
| 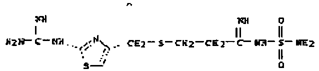 | **MERGETPA** | 77102-28-4 | Thrombinhemmer |
| 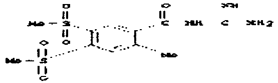 | **BCX 1812, Peramivir** | 330600-85-6 | Neuramidase-Hemmer (Influenza) |
| 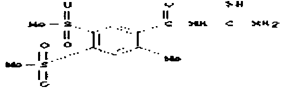 | **Apogastine, Famogast** | 76824-35-6 | Protonenpumenhemmer |
| 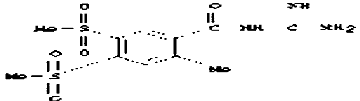 | | 65113-67-9 | |
| 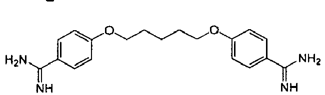 | **Pentamidin** | 100-33-4 | Antiprotozoikum, Antiinfektivum |

## Patentansprüche

1. Prodrug umfassend eine die allgemeine Formel (I) oder (II) aufweisende Teilstruktur 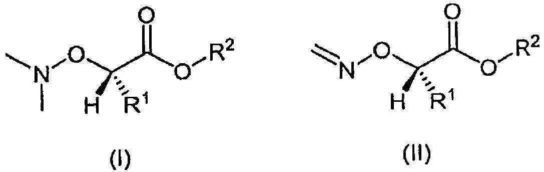 wobei R¹ und R² Wasserstoff, Alkyl- oder Arylreste sind,
**dadurch gekennzeichnet, dass**
die vom Prodrug umfasste Teilstruktur Bestandteil eines *N*-Oxids, eins Nitrons, eines Diazeniumdiolates (NONOat), einer Hydroxamsäure, eines Amidoxims (*N*-Hydroxyamidins), eines *N*-Hydroxyamidinohydrazons oder eines *N*-Hydroxyguanidins ist, und dass das Prodrug zu einem Arzneistoff metabolisiert wird, der ausgewählt ist aus Pentamidin, Dabigatran, BSF 411693 (Abbott), Idazoxanhydrochlorid, Irbesartan, Linogliride, Lofexidinehydrochloride, Tetrahydrozolinhydrochlorid, Tolazolin, Xylometazolin-hydrochlorid, Pentamidinisethionat, Taribavirin, Thiamin (Vitamin B1), Bosentan, Dibrompropamidinisetionat, Hydroxystilbamidmisethionate, Sibrafiban, Orbofiban, Xemilofiban, Argatroban, Ximelagatran, Melagatran, 2-Piperidinsäure, Orbofibanazetat, Epinastin (Relestat), RO 43-8857, AB1 (Chlorambucil, Analoga), AMG-126737, AY-0068, B-623, BABIM, BIBT-986 (Boehringer Ingelheim), CI-1031 (Firma: Biosciences), CJ-1332 (Firma: Curacyte), CJ-463 (Firma: Curacyte), CJ-672 (Firma: Curacyte), CT50728 (Portoila Pharmaceuticals), CVS-3983, DX-9065a, Lamifiban (Roche), LB-30870 (Firma: LG LifeSciences Ltd), LY-178550 (Firma: Lilly), PHA-927F und Analoga, RO-44-3888 (Roche), Sepimostat, FUT-187 (Torii), Viramidin (Ribapharm), WX-FX4 (Wilex), YM-60828 (Yamanouchi Pharmaceutical Co. Ltd), ZK-807191 (Berlex Biosciences), NAPAP (SR 25477), BIIL 315 (Boehringer Ingelheim), BIIL 260 (Boehringer Ingelheim), BIIL 284/260 (Boehringer Ingelheim), Tanogitran, Moxilubant, Stilbamidine, Panamidine, Fradafiban, Diminazene, Roxifiban, Furamidine, PD0313052, PHA 927F, PHA 798, Fidexabane, Otamixaban, Thromstop (Thrombstop), Zanamivir, Amiloridhydrochlorid, Anagrelidhydrochlorid, Proguanil, Cimetidin, Clonidinhydrochlorid, Guanoxa, Peramivir, Romifidine, Tirapazamine, Tizanidine, Tolo-nidinnitrat, Metformine, Diminazen, Debrisoquin, Sulfamethazine, Eptifibatide, Famotidine, Bayer-Arzneistoff (CAS No. 25836-74-2), Streptomycin, Nafamostat, FUT-175, Inogatran, Guanethidin (Thilodi-gon), 3DP-10017, APC-366, CVS-1123, Diphenyphosphonatderivat (CAS No. 5945-33-5), E-64, FOY-305, MBGB, MIBG, RWJ-422521, Synthalin, WX-293, WX-340, BMS-189090, JTV-803 (Japan Tabacco), Napsagatran, Ismelin, Tan 1057A, Hydikal, Phenformix (Retardo), Netropsin (Sinanomycin), BIIB 722 (Sabiporide), Guanadrel, Deoxyspergualin, BMS 262084, Siamformet (Orabet), PPACK (Pebac), MERGETPA (Plummer's Carboxypeptidase Inhibitor), Peramivir, Famotidine, Zaltidine und Orbofiban.

2. Prodrug nach einem der vorhergehenden Ansprüche,
**dadurch gekennzeichnet, dass**
das Prodrug bzw. der entsprechende Arzneistoff aus der Gruppe ausgewählt ist, die Proteasehemmstoffe, DNA- und RNA-interkalierende Verbindungen, Hemmstoffe viraler Enzyme und N-Methyl-D-Aspartat-Rezeptor-Antagonisten umfasst.

3. Prodrug nach einem der Ansprüche 1 oder 2,
**dadurch gekennzeichnet, dass**
die Teilstruktur die allgemeine Formel IIa oder IIb 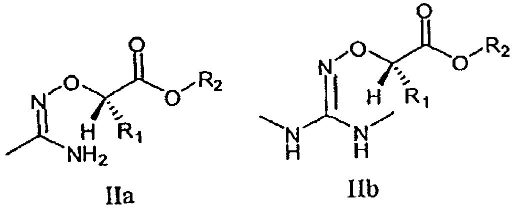 aufweist.

4. Prodrug nach Anspruch 3,
**dadurch gekennzeichnet, dass**
das Prodrug ein Prodrug zu einem Arzneistoff ist, wobei die Teilstruktur der allgemeinen Formel IIa nach Metabolisierung des Prodrugs zum Arzneistoff eine die Formel 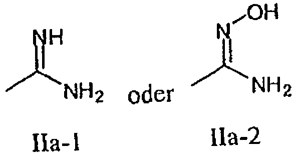 und die Teilstruktur der allgemeinen Formel IIb nach Metabolisierung des Prodrugs zum Arzneistoff eine die Formel 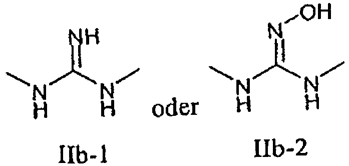 aufweisende Struktur besitzt.

5. Verwendung einer die allgemeine Formel (I) oder (II) ausbildende Teilstruktur 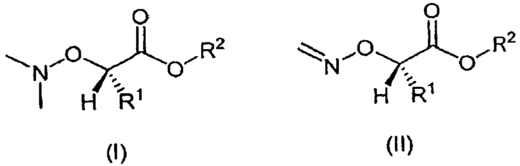 als Bestandteil der Gesamtstruktur eines Prodrugs, welches ein Prodrug zu einem Arzneistoff ist, wobei R¹ und R² Wasserstoff, Alkyl- oder Arylreste sind und der Arzneistoff ausgewählt ist aus Pentamidin, Dabigatran, BSF 411693 (Abbott), Idazoxanhydrochlorid, Irbesartan, Linogliride, Lofexidinehydrochloride, Tetrahydrozolinhydrochlorid, Tolazolin, Xylometazolin-hydrochlorid, Pentamidinisethionat, Taribavirin, Thiamin (Vitamin B1), Bosentan, Dibrompropamidinisethionat, Hydroxystilbamidmisethionate, Sibrafiban, Orbofiban, Xemilofiban, Argatroban, Ximelagatran, Melagatran, 2-Piperidinsäure, Orbofibanazetat, Epinastin (Relestat), RO 43-8857, AB1 (Chlorambucil, Analoga), AMG-126737, AY-0068, B-623, BABIM, BIBT-986 (Boehringer Ingelheim), CI-1031 (Firma: Biosciences), CJ-1332 (Firma: Curacyte), CJ-463 (Firma: Curacyte), CJ-672 (Firma: Curacyte), CT50728 (Portoila Pharmaceuticals), CVS-3983, DX-9065a, Lamifiban (Roche), LB-30870 (Firma: LG LifeSciences Ltd), LY-178550 (Firma: Lilly), PHA-927F und Analoga, RO-44-3888 (Roche), Sepimostat, FUT-187 (Torii), Viramidin (Ribapharm), WX-FX4 (Wilex), YM-60828 (Yamanouchi Pharmaceutical Co. Ltd), ZK-807191 (Berlex Biosciences), NAPAP (SR 25477), BIIL 315 (Boehringer Ingelheim), BIIL 260 (Boehringer Ingelheim), BIIL 284/260 (Boehringer Ingelheim), Tanogitran, Moxilubant, Stilbamidine, Panamidine, Fradafiban, Diminazene, Roxifiban, Furamidine, PD0313052, PHA 927F, PHA 798, Fidexabane, Otamixaban, Thromstop (Thrombstop), Zanamivir, Amiloridhydrochlorid, Anagrelidhydrochlorid, Proguanil, Cimetidin, Clonidinhydrochlorid, Guanoxa, Peramivir, Romifidine, Tirapazamine, Tizanidine, Tolo-nidinnitrat, Metformine, Diminazen, Debrisoquin, Sulfamethazine, Eptifibatide, Famotidine, Bayer-Arzneistoff (CAS No. 25836-74-2), Streptomycin, Nafamostat, FUT-175, Inogatran, Guanethidin (Thilodi-gon), 3DP-10017, APC -366, CVS-1123, Diphenyphosphonatderivat (CAS No. 5945-33-5), E-64, FOY-305, MBGB, MIBG, RWJ-422521, Synthalin, WX-293, WX-340, BMS-189090, JTV-803 (Japan Tabacco), Napsagatran, Ismelin, Tan 1057A, Hydikal, Phenformix (Retardo), Netropsin (Sinanomycin), BIIB 722 (Sabiporide), Guanadrel, Deoxyspergualin, BMS 262084, Siamformet (Orabet), PPACK (Pebac), MERGETPA (Plummer's Carboxypeptidase Inhibitor), Peramivir, Famotidine, Zaltidine, Orbofiban und Oktabin.

6. Verwendung nach Anspruch 5, wobei die Teilstruktur die allgemeine Formel (II) aufweist, diese Teil einer übergeordneten Teilstruktur IIa bzw. IIb 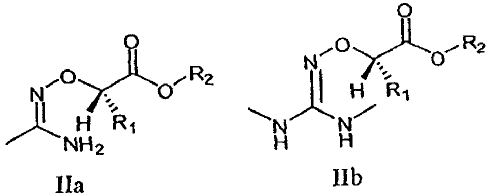 als Ersatz einer Amidin- bzw. Guanidin-Gruppe eines Arzneistoffes zur Verbesserung der Löslichkeit, oralen Bioverfügbarkeit, Blut-Hirn-Schrankengängigkeit, des Geschmacks und/oder der physikalisch-chemischen Stabilität.

7. Verwendung nach Anspruch 6, wobei das Prodrug ein Prodrug zu einem Arzneistoff ist, der die gleiche Struktur wie das Prodrug aufweist, abgesehen davon, dass er statt der übergeordneten Teilstruktur IIa eine der Teilstrukturen IIa-1 oder IIa-2 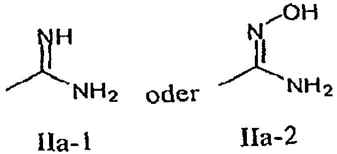 bzw. statt der übergeordneten Teilstruktur IIb eine der Teilstrukturen IIb-1 oder IIb-2 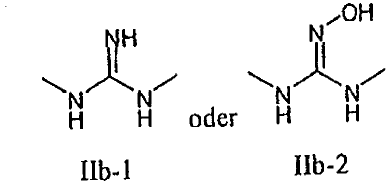 aufweist.

8. Verwendung nach Anspruch 6 oder 7 zur Aktivierung des Arzneistoffs durch Peptidylglycin α- Amidierende Monooxygenase (PAM).

9. Verwendung nach einem der Ansprüche 5 bis 8,
**dadurch gekennzeichnet, dass**
die Teilstruktur Bestandteil, eines *N*-Oxids, eines Nitrons, eines Diazeniumdiolates (NONOat), einer Hydroxamsäure, eines Amidoxims (*N-*Hydroxyamidins), eines
*N*-Hydroxyamidinohydrazons oder eines *N*-Hydroxyguanidins ist.

10. Verfahren zur Einschleusung eines Arzneistoffes, umfassend eine freie Amidin- oder Guanidinfunktion in den PAM-Aktivierungsweg, umfassend das Herstellen eines Prodrugs des Arzneistoffes nach einem der Ansprüche 1 bis 5, wobei das Verfahren nicht die therapeutische oder chirurgische Behandlung des menschlichen oder tierischen Körpers betrifft.

11. Verwendung eines Prodrugs nach einem der Ansprüche 1 bis 5 zur Herstellung eines Arzneistoffes.

12. Prodrug nach einem der Ansprüche 1 bis 5,
**dadurch gekennzeichnet, dass**
der Arzneistoff bzw. das Prodrug aus der Gruppe der Proteasehemmstoffe, der DNA- und RNA-interkalierenden Verbindungen, der Hemmstoffe viraler Enzyme und der N-Methyl-D-Aspartat-Rezeptor-Antagonisten ausgewählt ist.

13. Prodrug nach Anspruch 1 bis 5, oder 12, wobei die Verwendung eine Verwendung zur Prophylaxe und/oder Therapie der viszeralen und/oder kutanen Leishmaniose, der Trypanosomiasis, der 2. Phase der Trypanosomiasis oder der durch Pneumocystis carinii verursachten Pneumonie, zur Wachstumshemmung maligner Tumoren, zur Blutgerinnungshemmung, zur Blutdrucksenkung, zur Neuroprotektion oder zur Bekämpfung von viralen Infektionen, einschließlich Influenza und HIV-Infektionen ist.

## Claims

1. A prodrug, comprising a partial structure having the general formula (I) or (II) 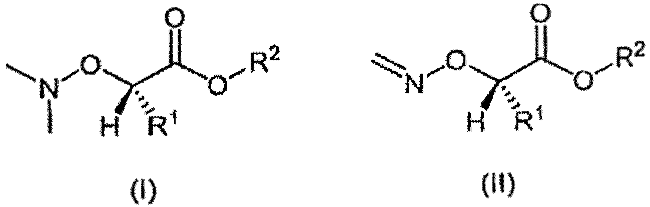 wherein R¹ and R² are hydrogen, alkyl, or aryl radicals,
**characterized in that**
the partial structure comprised by the prodrug is a component of an N-oxide, a nitron, a diazeniumdiolate (NONOat), a hydroxamic acid, an amidoxime (N-hydroxyamidin), an N-hydroxyamidinohydrazone, or an N-hydroxyguanidine, and **in that** the prodrug is metabolized to a drug substance selected from pentamidine, dabigatran, BSF 411693 (Abbott), idazoxan hydrochloride, irbesartan, linogliride, lofexidine hydrochloride, tetrahydrozoline hydrochloride, tolazoline, xylometazoline hydrochloride, pentamidine isethionate, taribavirin, thiamine (vitamin B1), bosentan, dibrompropamidine isethionate, hydroxystilbamidinic isethionate, sibrafiban, orbofiban, xemilofiban, argatroban, ximelagatran, melagatran, 2-piperidinic acid, orbofiban acetate, epinastine (Relestat), RO 43-8857, AB1 (Chlorambucil, analogues), AMG-126737, AY-0068, B-623, BABIM, BIBT-986 (Boehringer Ingelheim), CI-1031 (company: Biosciences) CJ-1332 (company: Curacyte), CJ-463 (company: Curacyte), CJ-672 (company: Curacyte), CT50728 (Portoila Pharmaceuticals), CVS-3983, DX-9065a, Lamifiban (Roche), LB-30870 (company: LG LifeSciences Ltd), LY-178550 (company: Lilly), PHA-927F and analogues, RO-44-3888 (Roche), sepimostat, FUT-187 (Torii), viramidine (Ribapharm), WX-FX4 (Wilex), YM-60828 (Yamanouchi Pharmaceutical Co. Ltd), ZK-807191 (Berlex Biosciences), NAPAP (SR 25477), BIIL 315 (Boehringer Ingelheim), BIIL 260 (Boehringer Ingelheim), BIIL 284/260 (Boehringer Ingelheim), tanogitran, moxilubant, stilbamidine, panamidine, fradafiban, diminazene, roxifiban, furamidine, PD0313052, PHA 927F, PHA 798, fidexaban, otamixaban, thromstop (Thrombstop), zanamivir, amiloride hydrochloride, anagrelide hydrochloride, proguanil, cimetidine, clonidine hydrochloride, guanoxan, peramivir, romifidine, tirapazamine, tizanidine, tolonidine nitrate, metformin, diminazene, debrisoquine, sulfamethazine, eptifibatide, famotidine, Bayer drug substance (CAS No. 25836-74-2), streptomycin, nafamostat, FUT-175, inogatran, guanethidine (Thilodigon), 3DP-10017, APC-366, CVS-1123, diphenyl phosphonate derivative (CAS No. 5945-33-5), E-64, FOY-305, MBGB, MIBG, RWJ-422521, Synthalin, WX-293, WX-340, BMS-189090, JTV-803 (Japan Tabacco), napsagatran, ismelin, Tan 1057A, Hydikal, Phenformix (Retardo), netropsin (Sinanomycin), BIIB 722 (sabiporide), guanadrel, deoxyspergualin, BMS 262084, Siamformet (Orabet), PPACK (Pebac), MERGETPA (Plummer's carboxypeptidase inhibitor), peramivir, famotidine, zaltidine and orbofiban.

2. The prodrug according to one of the preceding claims,
**characterized in that**
the prodrug respectively the corresponding drug substance is selected from the group comprising protease inhibitors, DNA- and RNA-intercalating compounds, inhibitors of viral enzymes and N-methyl-D-aspartate receptor antagonists.

3. The prodrug according to one of claims 1 or 2,
**characterized in that**
the partial structure has the general formula IIa or IIb

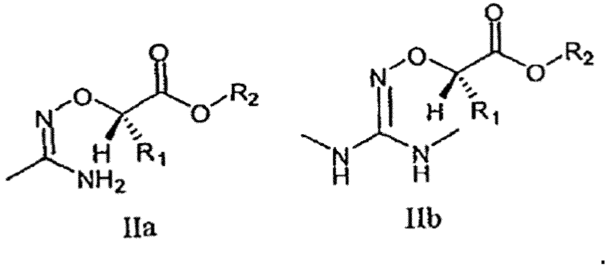

4. The prodrug according to claim 3,
**characterized in that**
the prodrug is a prodrug for a drug substance, wherein the partial structure of the general formula IIa, after metabolization of the prodrug to the drug substance, has a structure comprising the formula 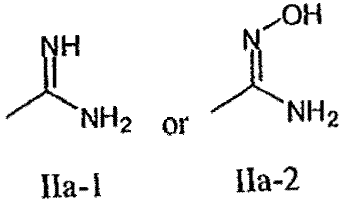 and the partial structure of the general formula IIb, after metabolization of the prodrug to the drug substance, has a structure comprising the formula

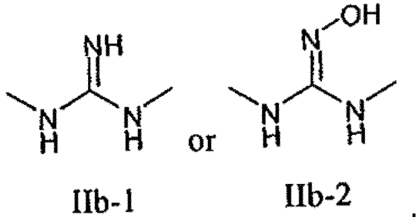

5. Use of a partial structure forming the general formula (I) or (II) 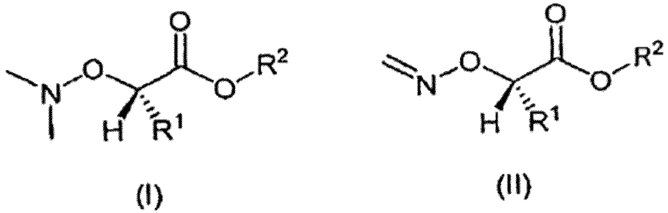 as a component of the overall structure of a prodrug which is a prodrug for a drug substance, wherein R¹ and R² are hydrogen, alkyl, or aryl radicals and the drug substance is selected from pentamidine, dabigatran, BSF 411693 (Abbott), idazoxan hydrochloride, irbesartan, linogliride, lofexidine hydrochloride, tetrahydrozoline hydrochloride, tolazoline, xylometazoline hydrochloride, pentamidine isethionate, taribavirin, thiamine (vitamin B1), bosentan, dibrompropamidine isethionate, hydroxystilbamidinic isethionate, sibrafiban, orbofiban, xemilofiban, argatroban, ximelagatran, melagatran, 2-piperidinic acid, orbofiban acetate, epinastine (Relestat), RO 43-8857, AB1 (Chlorambucil, analogues), AMG-126737, AY-0068, B-623, BABIM, BIBT-986 (Boehringer Ingelheim), CI-1031 (company: Biosciences) CJ-1332 (company: Curacyte), CJ-463 (company: Curacyte), CJ-672 (company: Curacyte), CT50728 (Portoila Pharmaceuticals), CVS-3983, DX-9065a, Lamifiban (Roche), LB-30870 (company: LG LifeSciences Ltd), LY-178550 (company: Lilly), PHA-927F and analogues, RO-44-3888 (Roche), sepimostat, FUT-187 (Torii), viramidine (Ribapharm), WX-FX4 (Wilex), YM-60828 (Yamanouchi Pharmaceutical Co. Ltd), ZK-807191 (Berlex Biosciences), NAPAP (SR 25477), BIIL 315 (Boehringer Ingelheim), BIIL 260 (Boehringer Ingelheim), BIIL 284/260 (Boehringer Ingelheim), tanogitran, moxilubant, stilbamidine, panamidine, fradafiban, diminazene, roxifiban, furamidine, PD0313052, PHA 927F, PHA 798, fidexaban, otamixaban, thromstop (Thrombstop), zanamivir, amiloride hydrochloride, anagrelide hydrochloride, proguanil, cimetidine, clonidine hydrochloride, guanoxan, peramivir, romifidine, tirapazamine, tizanidine, tolonidine nitrate, metformin, diminazene, debrisoquine, sulfamethazine, eptifibatide, famotidine, Bayer drug substance (CAS No. 25836-74-2), streptomycin, nafamostat, FUT-175, inogatran, guanethidine (Thilodigon), 3DP-10017, APC-366, CVS-1123, diphenyl phosphonate derivative (CAS No. 5945-33-5), E-64, FOY-305, MBGB, MIBG, RWJ-422521, Synthalin, WX-293, WX-340, BMS-189090, JTV-803 (Japan Tabacco), napsagatran, ismelin, Tan 1057A, Hydikal, Phenformix (Retardo), netropsin (Sinanomycin), BIIB 722 (sabiporide), guanadrel, deoxyspergualin, BMS 262084, Siamformet (Orabet), PPACK (Pebac), MERGETPA (Plummer's carboxypeptidase inhibitor), peramivir, famotidine, zaltidine, orbofiban and oktabin.

6. Use according to claim 5, wherein the partial structure has the general formula (II) which is part of a superordinate partial structure IIa respectively IIb 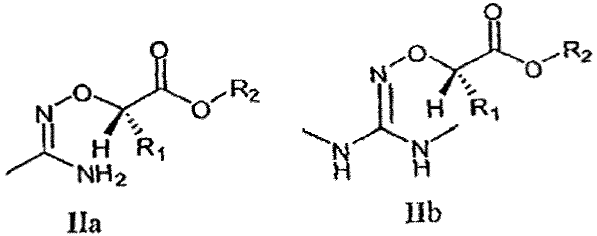 in the place of an amidine or guanidine group of a drug substance to improve solubility, oral bioavailability, blood-brain barrier crossing, the flavor, and/or physical-chemical stability.

7. Use according to claim 6, wherein the prodrug is a prodrug for a drug substance which has the same structure as the prodrug, except that instead of the superordinate partial structure IIa it comprises one of the partial structures IIa-1 or IIa-2 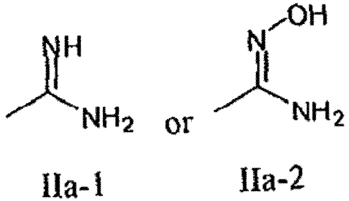 respectively instead of the superordinate partial structure IIb it comprises one of the partial structures IIb-1 or IIb-2

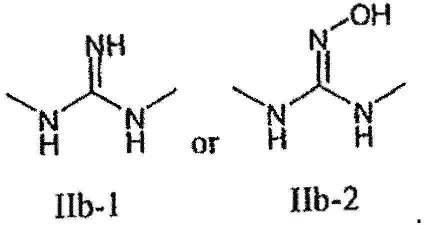

8. Use according to claim 6 or 7 for activating the drug substance by means of peptidylglycine α-amidating monooxygenase (PAM).

9. Use according to any one of claims 5 to 8,
**characterized in that**
the partial structure is a component of an N-oxide, a nitron, a diazeniumdiolate (NONOat), a hydroxamic acid, an amidoxime (N-hydroxyamidine), an N-hydroxyamidinohydrazone or an N-hydroxyguanidine.

10. A method for introducing of a drug substance, comprising a free amidine or guanidine function into the PAM activation path, comprising the production of a prodrug of the drug substance according to any one of claims 1 to 5, wherein the method does not concern the therapeutic or surgical treatment of the human or animal body.

11. Use of a prodrug according to any one of claims 1 to 5 for producing a drug substance.

12. The prodrug according to any one of claims 1 to 5,
**characterized in that**
the drug substance respectively the prodrug is selected from the group consisting of protease inhibitors, DNA- and RNA-intercalating compounds, inhibitors of viral enzymes, and N-methyl-D-aspartate receptor antagonists.

13. The prodrug according to claims 1 to 5, or 12, wherein the use is a use for the prophylaxis and/or therapy of visceral and/or cutaneous leishmaniasis, trypanosomiasis, phase 2 of trypanosomiasis or pneumonia caused by Pneumocystis carinii, for inhibiting the growth of malignant tumors, for inhibiting blood coagulation, for lowering blood pressure, for neuroprotection, or for combating viral infections, including influenza and HIV infections.

## Revendications

1. Promédicament contenant une structure partielle qui présente la formule (I) ou (II) 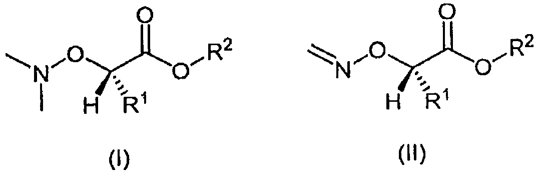 dans laquelle R₁ et R₂ sont de l'hydrogène ou des radicaux alkyle ou aryle,
**caractérisé en ce que** la structure partielle contenue dans le promédicament fait partie d'un oxyde d'azote, d'un nitron, d'un diolate de diazénium (NONOate), d'un acide hydroxamique, d'une amidoxime (N-hydroxyamidine), d'une N-hydroxyamidinohydrazone ou d'une N-hydroxyguanidine et **en ce que** le promédicament est métabolisé pour produire un médicament choisi parmi la pentamidine, le dabigatran, le BSF 411693 (Abbott), le chlorhydrate d'idazoxan, l'irbésartan, le linogliride, le chlorhydrate de loféxidine, le chlorhydrate de tétrahydrozoline, la tolazoline, le chlorhydrate de xylométazoline, l'iséthionate de pentamidine, la taribavirine, la thiamine (vitamine B1), le bosentan, l'iséthionate de dibromopropamidine, l'iséthionate d'hydroxystilbamidine, le sibafiban, l'orbofiban, le xémilofiban, l'argatroban, le ximélagatran, le mélagatran, l'acide 2-pipéridinique, l'acétate d'orbofiban, l'épinastine (Relestat), le RO 43-8857, l'AB1 (chlorambucil, analogues), l'AMG-126737, l'AY-0068, B-623, le BABIM, le BIBT-986 (Boehringer Ingelheim), le CI-1031 (laboratoire : Biosciences), le CJ-1332 (laboratoire : Curacyte), le CJ-463 (laboratoire : Curacyte), le CJ-672 (laboratoire : Curacyte), le CT50728 (Portoila Pharmaceuticals), le CVS-3983, le DX-9065a, le lamifiban (Roche), le LB-30870 (laboratoire : LG LifeSciences Ltd), le LY-178550 (laboratoire : Lilly), le PHA-927F et ses analogues, le RO-44-3888 (Roche), le sépimostat, le FUT-187 (Torii), la viramidine (Ribapharm), le WX-FX4 (Wilex), le YM-60828 (Yamanouchi Pharmaceutical Co. Ltd), le ZK-807191 (Berlex Biosciences), le NAPAP (SR 25477), le BIIL 315 (Boehringer Ingelheim), le BIIL 260 (Boehringer Ingelheim), le BIIL 284/260 (Boehringer Ingelheim), le tanogitran, le moxilubant, la stilbamidine, la panamidine, le fradafiban, le diminazène, le roxifiban, la furamidine, le PD0313052, le PHA 927F, le PHA 798, le fidexaban, l'otamixaban, le thromstop (Thrombstop), le zanamivir, le chlorhydrate d'amiloride, le chlorhydrate d'anagrélide, le proguanil, la cimétidine, le chlorhydrate de clonidine, le guanoxa, le péramivir, la romifidine, la tirapazamine, la tizanidine, le nitrate de tolonidine, la metformine, le diminazène, la débrisoquine, la sulfaméthazine, l'eptifibatide, la famotidine, un médicament de Bayer (N° CAS 25836-74-2), la streptomycine, le nafamostat, le FUT-175, l'inogatran, la guanéthidine (Thilodigon), le 3DP-10017, l'APC-366, le CVS-1123, un dérivé de diphénylphosphonate (N° CAS 5945-33-5), l'E-64, le FOY-305, le MBGB, le MIBG, le RWJ-422521, la synthaline, le WX-293, le WX-340, le BMS-189090, le JTV-803 (Japan Tabacco), le napsagatran, l'isméline, le TAN 1057A, l'Hydikal, le Phenformix (Retardo), la nétropsine (sinanomycine), le BIIB 722 (sabiporide), le guanadrel, la désoxyspergualine, le BMS 262084, le Siamformet (Orabet), la PPACK (Pebac), le MERGETPA (inhibiteur de carboxypeptidases de Plummer), le péramivir, la famotidine, la zaltidine et l'orbofiban.

2. Promédicament selon l'une des revendications précédentes, **caractérisé en ce que** le promédicament ou le médicament correspondant est choisi parmi le groupe comprenant des inhibiteurs de protéase, des composés s'intercalant dans l'ADN et l'ARN, des inhibiteurs d'enzymes virales et des antagonistes du récepteur du N-méthyl-D-aspartate.

3. Promédicament selon l'une des revendications 1 ou 2, **caractérisé en ce que** la structure partielle présente la formule générale IIa ou IIb

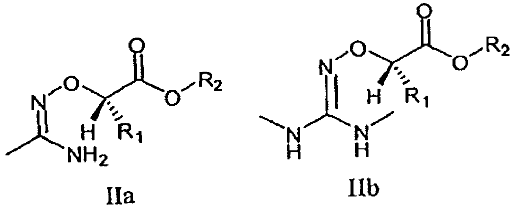

4. Promédicament selon la revendication 3, **caractérisé en ce qu'**il s'agit d'un promédicament pour un médicament dans lequel la structure partielle de formule générale IIa présente, après métabolisation du promédicament en médicament, une structure de formule 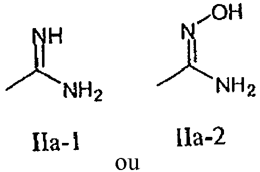 et la structure partielle de formule générale IIb présente, après métabolisation du promédicament en médicament, une structure de formule

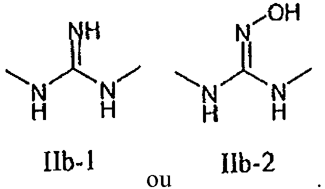

5. Utilisation d'une structure partielle formant la formule générale (I) ou (II) 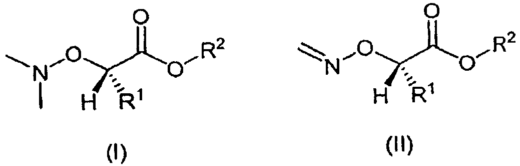 comme composante de la structure d'ensemble d'un promédicament qui est un promédicament pour un médicament, R₁ et R₂ étant de l'hydrogène ou des radicaux alkyle ou aryle et le médicament étant choisi parmi la pentamidine, le dabigatran, le BSF 411693 (Abbott), le chlorhydrate d'idazoxan, l'irbésartan, le linogliride, le chlorhydrate de loféxidine, le chlorhydrate de tétrahydrozoline, la tolazoline, le chlorhydrate de xylométazoline, l'iséthionate de pentamidine, la taribavirine, la thiamine (vitamine B1), le bosentan, l'iséthionate de dibrompropamidine, l'iséthionate d'hydroxystilbamidine, le sibafiban, l'orbofiban, le xémilofiban, l'argatroban, le ximélagatran, le mélagatran, l'acide 2-pipéridinique, l'acétate d'orbofiban, l'épinastine (Relestat), le RO 43-8857, l'AB1 (chlorambucil, analogues), l'AMG-126737, l'AY-0068, B-623, le BABIM, le BIBT-986 (Boehringer Ingelheim), le CI-1031 (laboratoire : Biosciences), 0-1332 (laboratoire : Curacyte), le CJ-463 (laboratoire : Curacyte), le CJ-672 (laboratoire : Curacyte), le CT50728 (Portoila Pharmaceuticals), le CVS-3983, le DX-9065a, le lamifiban (Roche), le LB-30870 (laboratoire : LG LifeSciences Ltd), le LY-178550 (laboratoire : Lilly), le PHA-927F et ses analogues, le RO-44-3888 (Roche), le sépimostat, le FUT-187 (Torii), la viramidine (Ribapharm), le WX-FX4 (Wilex), le YM-60828 (Yamanouchi Pharmaceutical Co. Ltd), le ZK-807191 (Berlex Biosciences), le NAPAP (SR 25477), le BIIL 315 (Boehringer Ingelheim), le BIIL 260 (Boehringer Ingelheim), le BIIL 284/260 (Boehringer Ingelheim), le tanogitran, le moxilubant, la stilbamidine, la panamidine, le fradafiban, le diminazène, le roxifiban, la furamidine, le PD0313052, le PHA 927F, le PHA 798, le fidexaban, l'otamixaban, le thromstop (Thrombstop), le zanamivir, le chlorhydrate d'amiloride, le chlorhydrate d'anagrélide, le proguanil, la cimétidine, le chlorhydrate de clonidine, le guanoxa, le péramivir, la romifidine, la tirapazamine, la tizanidine, le nitrate de tolonidine, la metformine, le diminazène, la débrisoquine, la sulfaméthazine, l'eptifibatide, la famotidine, un médicament de Bayer (N° CAS 25836-74-2), la streptomycine, le nafamostat, le FUT-175, l'inogatran, la guanéthidine (Thilodigon), le 3DP-10017, l'APC-366, le CVS-1123, un dérivé de diphénylphosphonate (N° CAS 5945-33-5), l'E-64, le FOY-305, le MBGB, le MIBG, le RWJ-422521, la synthaline, le WX-293, le WX-340, le BMS-189090, le JTV-803 (Japan Tabacco), le napsagatran, l'isméline, le TAN 1057A, l'Hydikal, le Phenformix (Retardo), la nétropsine (sinanomycine), le BIIB 722 (sabiporide), le guanadrel, la désoxyspergualine, le BMS 262084, le Siamformet (Orabet), la PPACK (Pebac), le MERGETPA (inhibiteur de carboxypeptidases de Plummer), le péramivir, la famotidine, la zaltidine, l'orbofiban et l'oktabine.

6. Utilisation selon la revendication 5, dans laquelle la structure partielle présente la formule générale (II) et cette partie d'une structure partielle supérieure IIa ou Ilb 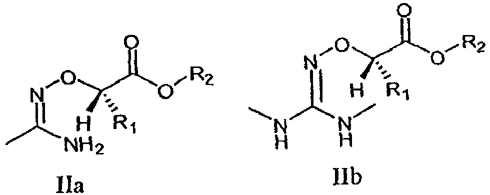 se substitue à un groupement amidine ou guanidine d'un médicament afin d'améliorer la solubilité, la biodisponibilité orale, le passage de la barrière hématoencéphalique, le goût et/ou la stabilité physicochimique.

7. Utilisation selon la revendication 6, dans laquelle le promédicament est un promédicament pour un médicament qui présente la même structure que le promédicament, mis à part le fait qu'au lieu de la structure partielle supérieure IIa, il présente l'une des structures partielles IIa-1 ou IIa-2 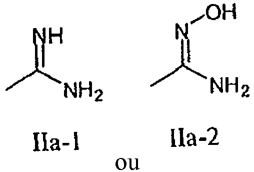 et au lieu de la structure partielle supérieure IIb, l'une des structures partielles IIb-1 ou IIb-2

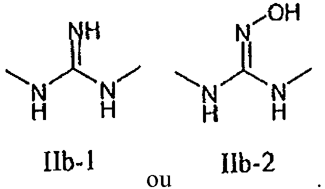

8. Utilisation selon la revendication 6 ou 7 pour l'activation du médicament par de la peptidylglycine monooxygénase α-amidante (PAM).

9. Utilisation selon l'une des revendications 5 à 8, **caractérisée en ce que** la structure partielle fait partie d'un oxyde d'azote, d'un nitron, d'un diolate de diazénium (NONOate), d'un acide hydroxamique, d'une amidoxime (N-hydroxyamidine), d'une N-hydroxyamidinohydrazone ou d'une N-hydroxyguanidine.

10. Procédé pour l'introduction d'un médicament contenant une fonction amidine ou guanidine libre dans la voie d'activation de la PAM, comprenant la fabrication d'un promédicament du médicament selon l'une des revendications 1 à 5, lequel procédé ne concerne pas le traitement thérapeutique ou chirurgical du corps humain ou animal.

11. Utilisation d'un promédicament selon l'une des revendications 1 à 5 pour la fabrication d'un médicament.

12. Promédicament selon l'une des revendications 1 à 5, **caractérisé en ce que** le promédicament ou le médicament correspondant est choisi parmi le groupe comprenant des inhibiteurs de protéase, des composés s'intercalant dans l'ADN et l'ARN, des inhibiteurs d'enzymes virales et des antagonistes du récepteur du N-méthyl-D-aspartate.

13. Promédicament selon les revendications 1 à 5 ou 12, dans lequel l'utilisation est une utilisation pour la prophylaxie et/ou le traitement de la leishmaniose viscérale et/ou cutanée, de la trypanosomiase, de la deuxième phase de la trypanosomiase ou de la pneumonie causée par *Pneumocystis carinii,* pour l'inhibition de la croissance de tumeurs malignes, pour l'anticoagulation, pour la réduction de la tension artérielle, pour la neuroprotection ou pour lutter contre les infections virales, y compris la grippe et les infections par le VIH.
